# EUROPÄISCHE PATENTANMELDUNG

(11) **EP 1 734 366 A2**
(43) Veröffentlichungstag der Anmeldung: **20.12.2006**
(21) Anmeldenummer: 06015257.6
(22) Anmeldetag: 29.10.1999
(51) Int. Cl.: G01N 33/569, C07K 16/12

(54) **Neues Verfahren zum Nachweis von Säure-resistenten Mikroorganismen im Stuhl**

(30) Priorität: 29.10.1998 EP 98120517; 06.11.1998 EP 98120687
(62) Teilanmeldung aus: 99971515.4
(71) Anmelder: DakoCytomation Denmark A/S, DK - 2600 Glostrup (DK)
(72) Erfinder: Reiter, Christian, 85757 Karlsfeld (DE); Cullmann, Gerhard, 81371 München (DE); Friedrichs, Ulrike, 04317 Leipzig (DE); Heppner, Petra, 82049 Pullach (DE); Lakner, Meret, 80689 München (DE); Ringeis, Achim, 82166 Gräfelfing (DE)
(74) Vertreter: Eisenführ, Speiser & Partner

(57) **Zusammenfassung**

Die Erfindung betrifft ein Verfahren zum Nachweis einer Infektion eines Säugers mit einem Säure-resistenten Mikroorganismus, wobei man (a) eine Stuhlprobe des Säugers mit mindestens zwei verschiedenen monoklonalen Antikörpern, Fragmenten oder Derivaten davon oder Aptameren unter Bedingungen inkubiert, die eine Komplexbildung von Antigenen aus dem Säure-resistenten Mikroorganismus mit den Antikörpern, Fragmenten oder Derivaten davon oder den Aptameren erlauben, und wobei (aa) der erste monoklonale Antikörper oder das Fragment oder Derivat davon oder das erste Aptamer ein Epitop des ersten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; (ab) der zweite monoklonale Antikörper oder das Fragment oder Derivat davon oder das zweite Aptamer ein vom Epitop des ersten Antigens verschiedenes Epitop eines zweiten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert, wobei die Teile der Säuger gemäß (aa) und gemäß (ab) überlappen können und in der Summe im wesentlichen die Gesamtanzahl der infizierten Säuger ausmachen; und (b) die Bildung mindestens eines Antigen-Antikörperkomplexes oder Antigen-Aptamerkomplexes gemäß (aa) oder (ab) nachweist.

SEQUENZPROTOKOLL

<110> CONNEX GMBH

<120> Neues Verfahren zum Nachweis von Säure-resistenten
      Mikroorganismen im Stuhl

<130> C 1786 PCT

<140> PCT/EP99/08212
<141> 1999-10-29

<160> 64

<170> PatentIn Ver. 2.1

<210> 1
<211> 10
<212> PRT
<213> Künstliche Sequenz

<220>
<223> Beschreibung der künstlichen Sequenz: künstliche
      Sequenz

<400> 1
Gly Phe Ser Leu Ser Arg Tyr Ser Val His
  1               5                  10

<210> 2
<211> 16
<212> PRT
<213> Künstliche Sequenz

<220>
<223> Beschreibung der künstlichen Sequenz: künstliche
      Sequenz

<400> 2
Met Ile Trp Gly Gly Gly Ser Thr Asp Tyr Asn Ser Gly Leu Lys Ser
  1               5                  10                  15

<210> 3
<211> 12
<212> PRT
<213> Künstliche Sequenz

<220>
<223> Beschreibung der künstlichen Sequenz: künstliche
      Sequenz

<400> 3
Asn Met Gly Gly Arg Tyr Pro Asp Tyr Phe Asp Tyr
  1               5                  10

<210> 4
<211> 30
<212> DNA
<213> Künstliche Sequenz

<220>
<223> Beschreibung der künstlichen Sequenz: künstliche
      Sequenz

<400> 4

gggttctcat tatccagata tagtgtacac                                    30


<210> 5
<211> 48
<212> DNA
<213> Künstliche Sequenz

<220>
<223> Beschreibung der künstlichen Sequenz: künstliche
      Sequenz

<400> 5
atgatatggg gtggtggaag cacagactat aattcaggtc tcaaatcc                48


<210> 6
<211> 36
<212> DNA
<213> Künstliche Sequenz

<220>
<223> Beschreibung der künstlichen Sequenz: künstliche
      Sequenz

<400> 6
aatatggggg gtaggtaccc ggactacttt gactac                             36


<210> 7
<211> 15
<212> PRT
<213> Künstliche Sequenz

<220>
<223> Beschreibung der künstlichen Sequenz: künstliche
      Sequenz

<400> 7
Arg Ala Ser Lys Ser Val Ser Thr Ser Gly Tyr Ser Tyr Ile His
  1               5                  10                  15


<210> 8
<211> 7
<212> PRT
<213> Künstliche Sequenz

<220>
<223> Beschreibung der künstlichen Sequenz: künstliche
      Sequenz

<400> 8
Leu Ala Ser Asn Leu Glu Ser
  1               5


<210> 9
<211> 9
<212> PRT
<213> Künstliche Sequenz

<220>
<223> Beschreibung der künstlichen Sequenz: künstliche
      Sequenz

<400> 9
Gln His Ser Arg Glu Leu Pro Leu Thr

1               5


<210> 10
<211> 45
<212> DNA
<213> Künstliche Sequenz

<220>
<223> Beschreibung der künstlichen Sequenz: künstliche
      Sequenz

<400> 10
agggccagca agagtgtcag tacatctggc tatagttaca tacac                    45


<210> 11
<211> 21
<212> DNA
<213> Künstliche Sequenz

<220>
<223> Beschreibung der künstlichen Sequenz: künstliche
      Sequenz

<400> 11
cttgcatcca acctagaatc t                                              21


<210> 12
<211> 27
<212> DNA
<213> Künstliche Sequenz

<220>
<223> Beschreibung der künstlichen Sequenz: künstliche
      Sequenz

<400> 12
cagcacagta gggagcttcc gctcacg                                        27


<210> 13
<211> 10
<212> PRT
<213> Künstliche Sequenz

<220>
<223> Beschreibung der künstlichen Sequenz: künstliche
      Sequenz

<400> 13
Gly Phe Thr Phe Asn Ser Tyr Ala Met Tyr
  1               5                  10


<210> 14
<211> 19
<212> PRT
<213> Künstliche Sequenz

<220>
<223> Beschreibung der künstlichen Sequenz: künstliche
      Sequenz

<400> 14
Arg Ile Arg Ser Lys Ser Asp Asn Tyr Ala Thr Tyr Tyr Ala Asn Ser
  1               5                  10                  15

Val Lys Asp

<210> 15
<211> 13
<212> PRT
<213> Künstliche Sequenz

<220>
<223> Beschreibung der künstlichen Sequenz: künstliche
      Sequenz

<400> 15
Asp His Asp Lys Phe Pro Phe Tyr Tyr Ala Leu Asp Tyr
  1               5                  10

<210> 16
<211> 30
<212> DNA
<213> Künstliche Sequenz

<220>
<223> Beschreibung der künstlichen Sequenz: künstliche
      Sequenz

<400> 16
ggtttcacct tcaattccta tgccatgtac                                   30

<210> 17
<211> 57
<212> DNA
<213> Künstliche Sequenz

<220>
<223> Beschreibung der künstlichen Sequenz: künstliche
      Sequenz

<400> 17
cgcataagaa gtaaaagtga taattatgca acatattatg ccaattcagt gaaagac    57

<210> 18
<211> 39
<212> DNA
<213> Künstliche Sequenz

<220>
<223> Beschreibung der künstlichen Sequenz: künstliche
      Sequenz

<400> 18
gatcatgata agtttccttt ttactatgct ctggactac                         39

<210> 19
<211> 12
<212> PRT
<213> Künstliche Sequenz

<220>
<223> Beschreibung der künstlichen Sequenz: künstliche
      Sequenz

<400> 19

Thr Ala Ser Ser Ser Val Ser Ser Ser Tyr Leu His
1               5                   10

<210> 20
<211> 7
<212> PRT
<213> Künstliche Sequenz

<220>
<223> Beschreibung der künstlichen Sequenz: künstliche
      Sequenz

<400> 20
Ser Thr Ser Asn Leu Ala Ser
1               5

<210> 21
<211> 9
<212> PRT
<213> Künstliche Sequenz

<220>
<223> Beschreibung der künstlichen Sequenz: künstliche
      Sequenz

<400> 21
His Gln Tyr His Arg Ser Pro Pro Thr
1               5

<210> 22
<211> 36
<212> DNA
<213> Künstliche Sequenz

<220>
<223> Beschreibung der künstlichen Sequenz: künstliche
      Sequenz

<400> 22
actgccagct caagtgtgag ttccagttac ttgcac                                36

<210> 23
<211> 21
<212> DNA
<213> Künstliche Sequenz

<220>
<223> Beschreibung der künstlichen Sequenz: künstliche
      Sequenz

<400> 23
agcacttcca acctggcttc t                                                21

<210> 24
<211> 27
<212> DNA
<213> Künstliche Sequenz

<220>
<223> Beschreibung der künstlichen Sequenz: künstliche
      Sequenz

<400> 24

caccagtatc atcgttcccc accgacg                                    27

<210> 25
<211> 10
<212> PRT
<213> Künstliche Sequenz

<220>
<223> Beschreibung der künstlichen Sequenz: künstliche
      Sequenz

<400> 25
Gly Phe Thr Phe Ser Ser His Phe Met Ser
  1               5                  10


<210> 26
<211> 16
<212> PRT
<213> Künstliche Sequenz

<220>
<223> Beschreibung der künstlichen Sequenz: künstliche
      Sequenz

<400> 26
Ser Ile Ser Ser Gly Gly Asp Ser Phe Tyr Pro Asp Ser Leu Lys Gly
  1               5                  10                  15


<210> 27
<211> 9
<212> PRT
<213> Künstliche Sequenz

<220>
<223> Beschreibung der künstlichen Sequenz: künstliche
      Sequenz

<400> 27
Asp Tyr Ser Trp Tyr Ala Leu Asp Tyr
  1               5


<210> 28
<211> 10
<212> PRT
<213> Künstliche Sequenz

<220>
<223> Beschreibung der künstlichen Sequenz: künstliche
      Sequenz

<400> 28
Gly Tyr Ala Phe Ser Thr Ser Trp Met Asn
  1               5                  10


<210> 29
<211> 17
<212> PRT
<213> Künstliche Sequenz

<220>
<223> Beschreibung der künstlichen Sequenz: künstliche
      Sequenz

<400> 29
Arg Ile Tyr Pro Gly Asp Gly Asp Thr Asn Tyr Asn Gly Lys Phe Lys
1               5                   10                  15

Gly

<210> 30
<211> 13
<212> PRT
<213> Künstliche Sequenz

<220>
<223> Beschreibung der künstlichen Sequenz: künstliche
      Sequenz

<400> 30
Glu Asp Ala Tyr Tyr Ser Asn Pro Tyr Ser Leu Asp Tyr
1               5                   10

<210> 31
<211> 30
<212> DNA
<213> Künstliche Sequenz

<220>
<223> Beschreibung der künstlichen Sequenz: künstliche
      Sequenz

<400> 31
ggctacgcat tcagtacctc ctggatgaac                                        30

<210> 32
<211> 51
<212> DNA
<213> Künstliche Sequenz

<220>
<223> Beschreibung der künstlichen Sequenz: künstliche
      Sequenz

<400> 32
cggatttatc ctggagatgg agatactaac tacaatggga agttcaaggg c               51

<210> 33
<211> 39
<212> DNA
<213> Künstliche Sequenz

<220>
<223> Beschreibung der künstlichen Sequenz: künstliche
      Sequenz

<400> 33
gaggatgcct attatagtaa cccctatagt ttggactac                              39

<210> 34
<211> 30
<212> DNA
<213> Künstliche Sequenz

<220>
<223> Beschreibung der künstlichen Sequenz: künstliche

Sequenz

<400> 34
ggattcactt tcagtagcca tttcatgtct                                        30


<210> 35
<211> 48
<212> DNA
<213> Künstliche Sequenz

<220>
<223> Beschreibung der künstlichen Sequenz: künstliche
      Sequenz

<400> 35
tccattagta gtggtggtga cagtttctat ccagacagtc tgaagggc                    48


<210> 36
<211> 27
<212> DNA
<213> Künstliche Sequenz

<220>
<223> Beschreibung der künstlichen Sequenz: künstliche
      Sequenz

<400> 36
gactactctt ggtatgcttt ggactac                                           27


<210> 37
<211> 11
<212> PRT
<213> Künstliche Sequenz

<220>
<223> Beschreibung der künstlichen Sequenz: künstliche
      Sequenz

<400> 37
Arg Ala Ser Gln Ser Ile Gly Thr Arg Ile His
  1               5                  10


<210> 38
<211> 7
<212> PRT
<213> Künstliche Sequenz

<220>
<223> Beschreibung der künstlichen Sequenz: künstliche
      Sequenz

<400> 38
Tyr Gly Ser Glu Ser Ile Ser
  1               5


<210> 39
<211> 9
<212> PRT
<213> Künstliche Sequenz

<220>
<223> Beschreibung der künstlichen Sequenz: künstliche
      Sequenz

<400> 39
Gln Gln Ser Asn Thr Trp Pro Leu Thr
1               5


<210> 40
<211> 11
<212> PRT
<213> Künstliche Sequenz

<220>
<223> Beschreibung der künstlichen Sequenz: künstliche
      Sequenz

<400> 40
His Ala Ser Gln Asn Ile Asn Val Trp Leu Ser
1               5                   10


<210> 41
<211> 7
<212> PRT
<213> Künstliche Sequenz

<220>
<223> Beschreibung der künstlichen Sequenz: künstliche
      Sequenz

<400> 41
Lys Ala Ser Asn Leu His Thr
1               5


<210> 42
<211> 9
<212> PRT
<213> Künstliche Sequenz

<220>
<223> Beschreibung der künstlichen Sequenz: künstliche
      Sequenz

<400> 42
Gln Gln Gly Arg Ser Tyr Pro Leu Thr
1               5


<210> 43
<211> 33
<212> DNA
<213> Künstliche Sequenz

<220>
<223> Beschreibung der künstlichen Sequenz: künstliche
      Sequenz

<400> 43
agggccagtc agagcattgg cacaagaata cac                                33


<210> 44
<211> 21
<212> DNA
<213> Künstliche Sequenz

<220>
<223> Beschreibung der künstlichen Sequenz: künstliche

Sequenz

<400> 44
tatggttctg agtctatctc t                                                21


<210> 45
<211> 27
<212> DNA
<213> Künstliche Sequenz

<220>
<223> Beschreibung der künstlichen Sequenz: künstliche
      Sequenz

<400> 45
caacaaagta atacctggcc gctcacg                                          27


<210> 46
<211> 33
<212> DNA
<213> Künstliche Sequenz

<220>
<223> Beschreibung der künstlichen Sequenz: künstliche
      Sequenz

<400> 46
catgccagtc agaacattaa tgtttggtta agc                                   33


<210> 47
<211> 21
<212> DNA
<213> Künstliche Sequenz

<220>
<223> Beschreibung der künstlichen Sequenz: künstliche
      Sequenz

<400> 47
aaggcttcca acttgcacac a                                                21


<210> 48
<211> 27
<212> DNA
<213> Künstliche Sequenz

<220>
<223> Beschreibung der künstlichen Sequenz: künstliche
      Sequenz

<400> 48
caacagggtc gaagttatcc tctcacg                                          27


<210> 49
<211> 333
<212> DNA
<213> Mus musculus

<220>
<221> CDS
<222> (1)..(333)

<400> 49

gac att gtg ctg aca cag tct cct gct tcc tta gct gta tct ctg ggg     48
Asp Ile Val Leu Thr Gln Ser Pro Ala Ser Leu Ala Val Ser Leu Gly
1               5                   10                  15

cag agg gcc acc atc tca tgc agg gcc agc aag agt gtc agt aca tct     96
Gln Arg Ala Thr Ile Ser Cys Arg Ala Ser Lys Ser Val Ser Thr Ser
            20                  25                  30

ggc tat agt tac ata cac tgg tac caa cag aaa cca gga cag cca ccc     144
Gly Tyr Ser Tyr Ile His Trp Tyr Gln Gln Lys Pro Gly Gln Pro Pro
        35                  40                  45

aaa ctc ctc atc ttt ctt gca tcc aac cta gaa tct ggg gtc cct gcc     192
Lys Leu Leu Ile Phe Leu Ala Ser Asn Leu Glu Ser Gly Val Pro Ala
    50                  55                  60

agg ttc agt ggc agt ggg tct ggg aca gac ttc acc ctc aac atc cat     240
Arg Phe Ser Gly Ser Gly Ser Gly Thr Asp Phe Thr Leu Asn Ile His
65                  70                  75                  80

cct gtg gag gag gag gat gct gca acc tat cac tgt cag cac agt agg     288
Pro Val Glu Glu Glu Asp Ala Ala Thr Tyr His Cys Gln His Ser Arg
                85                  90                  95

gag ctt ccg ctc acg ttc ggt gct ggg acc aag ctg gag ctg aaa         333
Glu Leu Pro Leu Thr Phe Gly Ala Gly Thr Lys Leu Glu Leu Lys
            100                 105                 110


<210> 50
<211> 111
<212> PRT
<213> Mus musculus

<400> 50
Asp Ile Val Leu Thr Gln Ser Pro Ala Ser Leu Ala Val Ser Leu Gly
1               5                   10                  15

Gln Arg Ala Thr Ile Ser Cys Arg Ala Ser Lys Ser Val Ser Thr Ser
            20                  25                  30

Gly Tyr Ser Tyr Ile His Trp Tyr Gln Gln Lys Pro Gly Gln Pro Pro
        35                  40                  45

Lys Leu Leu Ile Phe Leu Ala Ser Asn Leu Glu Ser Gly Val Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr His Cys Gln His Ser Arg
                85                  90                  95

Glu Leu Pro Leu Thr Phe Gly Ala Gly Thr Lys Leu Glu Leu Lys
            100                 105                 110


<210> 51
<211> 363
<212> DNA
<213> Mus musculus

<220>
<221> CDS
<222> (1)..(363)

<400> 51

gag gtg cag ctg ctc gag gag tca gga cct ggc ctg gtg gca ccc tca     48
Glu Val Gln Leu Leu Glu Glu Ser Gly Pro Gly Leu Val Ala Pro Ser
1               5                   10                  15

cag agc ctg tcc atc aca tgc act gtc tct ggg ttc tca tta tcc aga     96
Gln Ser Leu Ser Ile Thr Cys Thr Val Ser Gly Phe Ser Leu Ser Arg
            20                  25                  30

tat agt gta cac tgg gtt cgc cag cct cca gga aag ggt ctg gag tgg     144
Tyr Ser Val His Trp Val Arg Gln Pro Pro Gly Lys Gly Leu Glu Trp
        35                  40                  45

ctg gga atg ata tgg ggt ggt gga agc aca gac tat aat tca ggt ctc     192
Leu Gly Met Ile Trp Gly Gly Gly Ser Thr Asp Tyr Asn Ser Gly Leu
    50                  55                  60

aaa tcc aga ctg agc atc agc aac gac aac tcc aag agc caa gtt ttc     240
Lys Ser Arg Leu Ser Ile Ser Asn Asp Asn Ser Lys Ser Gln Val Phe
65                  70                  75                  80

tta aaa atg aac agt ctg caa act gat gac aca gcc att tac tac tgt     288
Leu Lys Met Asn Ser Leu Gln Thr Asp Asp Thr Ala Ile Tyr Tyr Cys
                85                  90                  95

gcc aga aat atg ggg ggt agg tac ccg gac tac ttt gac tac tgg ggc     336
Ala Arg Asn Met Gly Gly Arg Tyr Pro Asp Tyr Phe Asp Tyr Trp Gly
            100                 105                 110

caa ggc acc act ctc aca gtc tcc tca                                 363
Gln Gly Thr Thr Leu Thr Val Ser Ser
        115                 120


<210> 52
<211> 121
<212> PRT
<213> Mus musculus

<400> 52
Glu Val Gln Leu Leu Glu Glu Ser Gly Pro Gly Leu Val Ala Pro Ser
1               5                   10                  15

Gln Ser Leu Ser Ile Thr Cys Thr Val Ser Gly Phe Ser Leu Ser Arg
            20                  25                  30

Tyr Ser Val His Trp Val Arg Gln Pro Pro Gly Lys Gly Leu Glu Trp
        35                  40                  45

Leu Gly Met Ile Trp Gly Gly Gly Ser Thr Asp Tyr Asn Ser Gly Leu
    50                  55                  60

Lys Ser Arg Leu Ser Ile Ser Asn Asp Asn Ser Lys Ser Gln Val Phe
65                  70                  75                  80

Leu Lys Met Asn Ser Leu Gln Thr Asp Asp Thr Ala Ile Tyr Tyr Cys
                85                  90                  95

Ala Arg Asn Met Gly Gly Arg Tyr Pro Asp Tyr Phe Asp Tyr Trp Gly
            100                 105                 110

Gln Gly Thr Thr Leu Thr Val Ser Ser
        115                 120


<210> 53
<211> 324
<212> DNA

<213> Mus musculus

<220>
<221> CDS
<222> (1)..(324)

<400> 53
gag ctc gtg ctc acc cag tct cca aca atc atg tct gca tct cta ggg     48
Glu Leu Val Leu Thr Gln Ser Pro Thr Ile Met Ser Ala Ser Leu Gly
1               5                   10                  15

gaa cgg gtc acc atg acc tgc act gcc agc tca agt gtg agt tcc agt     96
Glu Arg Val Thr Met Thr Cys Thr Ala Ser Ser Ser Val Ser Ser Ser
            20                  25                  30

tac ttg cac tgg tac cag cag aag cca gga tcc tcc ccc aaa ctc tgg     144
Tyr Leu His Trp Tyr Gln Gln Lys Pro Gly Ser Ser Pro Lys Leu Trp
        35                  40                  45

att tat agc act tcc aac ctg gct tct gga gtc cca gta cgc ttc agt     192
Ile Tyr Ser Thr Ser Asn Leu Ala Ser Gly Val Pro Val Arg Phe Ser
    50                  55                  60

ggc agt ggg tct gtg acc tct tac tct ctc aca atc agc agc atg gag     240
Gly Ser Gly Ser Val Thr Ser Tyr Ser Leu Thr Ile Ser Ser Met Glu
65                  70                  75                  80

gct gaa gat gct gcc act tat tat tgc cac cag tat cat cgt tcc cca     288
Ala Glu Asp Ala Ala Thr Tyr Tyr Cys His Gln Tyr His Arg Ser Pro
                85                  90                  95

ccg acg ttc ggt gga ggc acc aag ctg gaa atc aaa                     324
Pro Thr Phe Gly Gly Gly Thr Lys Leu Glu Ile Lys
            100                 105


<210> 54
<211> 108
<212> PRT
<213> Mus musculus

<400> 54
Glu Leu Val Leu Thr Gln Ser Pro Thr Ile Met Ser Ala Ser Leu Gly
1               5                   10                  15

Glu Arg Val Thr Met Thr Cys Thr Ala Ser Ser Ser Val Ser Ser Ser
            20                  25                  30

Tyr Leu His Trp Tyr Gln Gln Lys Pro Gly Ser Ser Pro Lys Leu Trp
        35                  40                  45

Ile Tyr Ser Thr Ser Asn Leu Ala Ser Gly Val Pro Val Arg Phe Ser
    50                  55                  60

Gly Ser Gly Ser Val Thr Ser Tyr Ser Leu Thr Ile Ser Ser Met Glu
65                  70                  75                  80

Ala Glu Asp Ala Ala Thr Tyr Tyr Cys His Gln Tyr His Arg Ser Pro
                85                  90                  95

Pro Thr Phe Gly Gly Gly Thr Lys Leu Glu Ile Lys
            100                 105


<210> 55
<211> 375
<212> DNA

<213> Mus musculus

<220>
<221> CDS
<222> (1)..(375)

<400> 55
gag gtg cag ctg ctc gag gag tct ggg gga gga ttg gtc caa cct aca     48
Glu Val Gln Leu Leu Glu Glu Ser Gly Gly Gly Leu Val Gln Pro Thr
1               5                   10                  15

gga tca ttg aaa ctc tca tgt gcc gcc tct ggt ttc acc ttc aat tcc     96
Gly Ser Leu Lys Leu Ser Cys Ala Ala Ser Gly Phe Thr Phe Asn Ser
            20                  25                  30

tat gcc atg tac tgg gtc cgc cag gct cca gga aag ggt ttg gag tgg     144
Tyr Ala Met Tyr Trp Val Arg Gln Ala Pro Gly Lys Gly Leu Glu Trp
        35                  40                  45

gtt gct cgc ata aga agt aaa agt gat aat tat gca aca tat tat gcc     192
Val Ala Arg Ile Arg Ser Lys Ser Asp Asn Tyr Ala Thr Tyr Tyr Ala
    50                  55                  60

aat tca gtg aaa gac aga ctc acc atc tcc aga gat gat tca caa aac     240
Asn Ser Val Lys Asp Arg Leu Thr Ile Ser Arg Asp Asp Ser Gln Asn
65                  70                  75                  80

atg ctc tat ctg cag atg aac aac ctg aaa act gag gac aca gcc atg     288
Met Leu Tyr Leu Gln Met Asn Asn Leu Lys Thr Glu Asp Thr Ala Met
                85                  90                  95

tat tac tgt gtg aga gat cat gat aag ttt cct ttt tac tat gct ctg     336
Tyr Tyr Cys Val Arg Asp His Asp Lys Phe Pro Phe Tyr Tyr Ala Leu
            100                 105                 110

gac tac tgg ggt cca gga acc tta gtc acc gtc tcc tca                 375
Asp Tyr Trp Gly Pro Gly Thr Leu Val Thr Val Ser Ser
        115                 120                 125


<210> 56
<211> 125
<212> PRT
<213> Mus musculus

<400> 56
Glu Val Gln Leu Leu Glu Glu Ser Gly Gly Gly Leu Val Gln Pro Thr
1               5                   10                  15

Gly Ser Leu Lys Leu Ser Cys Ala Ala Ser Gly Phe Thr Phe Asn Ser
            20                  25                  30

Tyr Ala Met Tyr Trp Val Arg Gln Ala Pro Gly Lys Gly Leu Glu Trp
        35                  40                  45

Val Ala Arg Ile Arg Ser Lys Ser Asp Asn Tyr Ala Thr Tyr Tyr Ala
    50                  55                  60

Asn Ser Val Lys Asp Arg Leu Thr Ile Ser Arg Asp Asp Ser Gln Asn
65                  70                  75                  80

Met Leu Tyr Leu Gln Met Asn Asn Leu Lys Thr Glu Asp Thr Ala Met
                85                  90                  95

Tyr Tyr Cys Val Arg Asp His Asp Lys Phe Pro Phe Tyr Tyr Ala Leu
            100                 105                 110

Asp Tyr Trp Gly Pro Gly Thr Leu Val Thr Val Ser Ser

115                    120                    125

<210> 57
<211> 321
<212> DNA
<213> Mus musculus

<220>
<221> CDS
<222> (1)..(321)

<400> 57
gac atc ttg ctg act cag tct cca gcc atc ctg tct gtg agt cca gga     48
Asp Ile Leu Leu Thr Gln Ser Pro Ala Ile Leu Ser Val Ser Pro Gly
1               5                   10                  15

gaa aga gtc agt ttc tcc tgc agg gcc agt cag agc att ggc aca aga     96
Glu Arg Val Ser Phe Ser Cys Arg Ala Ser Gln Ser Ile Gly Thr Arg
            20                  25                  30

ata cac tgg tat caa caa aga aca aat ggt tct cca agg ctt ctc ata     144
Ile His Trp Tyr Gln Gln Arg Thr Asn Gly Ser Pro Arg Leu Leu Ile
        35                  40                  45

aag tat ggt tct gag tct atc tct ggg atc cct tcc agg ttt agt ggc     192
Lys Tyr Gly Ser Glu Ser Ile Ser Gly Ile Pro Ser Arg Phe Ser Gly
    50                  55                  60

agt gga tca ggg aca gat ttt agt ctt agc atc aac agt gtc gag tct     240
Ser Gly Ser Gly Thr Asp Phe Ser Leu Ser Ile Asn Ser Val Glu Ser
65                  70                  75                  80

gaa gat att gca gat tat tac tgt caa caa agt aat acc tgg ccg ctc     288
Glu Asp Ile Ala Asp Tyr Tyr Cys Gln Gln Ser Asn Thr Trp Pro Leu
                85                  90                  95

acg ttc ggt gct ggg acc aag ctg gag ctg aaa                         321
Thr Phe Gly Ala Gly Thr Lys Leu Glu Leu Lys
            100                 105


<210> 58
<211> 107
<212> PRT
<213> Mus musculus

<400> 58
Asp Ile Leu Leu Thr Gln Ser Pro Ala Ile Leu Ser Val Ser Pro Gly
1               5                   10                  15

Glu Arg Val Ser Phe Ser Cys Arg Ala Ser Gln Ser Ile Gly Thr Arg
            20                  25                  30

Ile His Trp Tyr Gln Gln Arg Thr Asn Gly Ser Pro Arg Leu Leu Ile
        35                  40                  45

Lys Tyr Gly Ser Glu Ser Ile Ser Gly Ile Pro Ser Arg Phe Ser Gly
    50                  55                  60

Ser Gly Ser Gly Thr Asp Phe Ser Leu Ser Ile Asn Ser Val Glu Ser
65                  70                  75                  80

Glu Asp Ile Ala Asp Tyr Tyr Cys Gln Gln Ser Asn Thr Trp Pro Leu
                85                  90                  95

Thr Phe Gly Ala Gly Thr Lys Leu Glu Leu Lys

100                 105

<210> 59
<211> 369
<212> DNA
<213> Mus musculus

<220>
<221> CDS
<222> (1)..(369)

<400> 59
gag gtg cag ctg ctc gag cag tct gga gct gag ctg gtg aag cct ggg      48
Glu Val Gln Leu Leu Glu Gln Ser Gly Ala Glu Leu Val Lys Pro Gly
1               5                   10                  15

gcc tca gtg aag att tcc tgc aag gct tct ggc tac gca ttc agt acc      96
Ala Ser Val Lys Ile Ser Cys Lys Ala Ser Gly Tyr Ala Phe Ser Thr
            20                  25                  30

tcc tgg atg aac tgg gtg aaa cag agg cct gga aag ggt ctt gag tgg      144
Ser Trp Met Asn Trp Val Lys Gln Arg Pro Gly Lys Gly Leu Glu Trp
        35                  40                  45

att gga cgg att tat cct gga gat gga gat act aac tac aat ggg aag      192
Ile Gly Arg Ile Tyr Pro Gly Asp Gly Asp Thr Asn Tyr Asn Gly Lys
    50                  55                  60

ttc aag ggc aag gcc aca ctg act gca gac aaa tcc tcc agc aca gcc      240
Phe Lys Gly Lys Ala Thr Leu Thr Ala Asp Lys Ser Ser Ser Thr Ala
65                  70                  75                  80

tac atg caa ctc aac agc ctg aca tct gag gac tct gcg gtc tac ttc      288
Tyr Met Gln Leu Asn Ser Leu Thr Ser Glu Asp Ser Ala Val Tyr Phe
                85                  90                  95

tgt gta aga gag gat gcc tat tat agt aac ccc tat agt ttg gac tac      336
Cys Val Arg Glu Asp Ala Tyr Tyr Ser Asn Pro Tyr Ser Leu Asp Tyr
            100                 105                 110

tgg ggt caa gga acc tca gtc acc gtc tcc tca                          369
Trp Gly Gln Gly Thr Ser Val Thr Val Ser Ser
        115                 120

<210> 60
<211> 123
<212> PRT
<213> Mus musculus

<400> 60
Glu Val Gln Leu Leu Glu Gln Ser Gly Ala Glu Leu Val Lys Pro Gly
1               5                   10                  15

Ala Ser Val Lys Ile Ser Cys Lys Ala Ser Gly Tyr Ala Phe Ser Thr
            20                  25                  30

Ser Trp Met Asn Trp Val Lys Gln Arg Pro Gly Lys Gly Leu Glu Trp
        35                  40                  45

Ile Gly Arg Ile Tyr Pro Gly Asp Gly Asp Thr Asn Tyr Asn Gly Lys
    50                  55                  60

Phe Lys Gly Lys Ala Thr Leu Thr Ala Asp Lys Ser Ser Ser Thr Ala
65                  70                  75                  80

Tyr Met Gln Leu Asn Ser Leu Thr Ser Glu Asp Ser Ala Val Tyr Phe
                85                  90                  95

Cys Val Arg Glu Asp Ala Tyr Tyr Ser Asn Pro Tyr Ser Leu Asp Tyr
            100                 105                 110

Trp Gly Gln Gly Thr Ser Val Thr Val Ser Ser
        115                 120

<210> 61
<211> 321
<212> DNA
<213> Mus musculus

<220>
<221> CDS
<222> (1)..(321)

<400> 61
gag ctc cag atg acc cag tct cca tcc agt ctg tct gca tcc ctt gga 48
Glu Leu Gln Met Thr Gln Ser Pro Ser Ser Leu Ser Ala Ser Leu Gly
1               5                   10                  15

gac aca att acc atc act tgc cat gcc agt cag aac att aat gtt tgg 96
Asp Thr Ile Thr Ile Thr Cys His Ala Ser Gln Asn Ile Asn Val Trp
            20                  25                  30

tta agc tgg tat cag cag aaa cca gga gat atc cct aaa cta ttg atc 144
Leu Ser Trp Tyr Gln Gln Lys Pro Gly Asp Ile Pro Lys Leu Leu Ile
        35                  40                  45

tat aag gct tcc aac ttg cac aca ggc gtc cca tca agg ttt agt ggc 192
Tyr Lys Ala Ser Asn Leu His Thr Gly Val Pro Ser Arg Phe Ser Gly
    50                  55                  60

agt gga tct gga aca ggt ttc aca tta gtc atc agc agc ctg cag cct 240
Ser Gly Ser Gly Thr Gly Phe Thr Leu Val Ile Ser Ser Leu Gln Pro
65                  70                  75                  80

gaa gac att gcc act tac tac tgt caa cag ggt cga agt tat cct ctc 288
Glu Asp Ile Ala Thr Tyr Tyr Cys Gln Gln Gly Arg Ser Tyr Pro Leu
                85                  90                  95

acg ttc ggt gct ggg acc aag ctg gag ctg aaa 321
Thr Phe Gly Ala Gly Thr Lys Leu Glu Leu Lys
            100                 105

<210> 62
<211> 107
<212> PRT
<213> Mus musculus

<400> 62
Glu Leu Gln Met Thr Gln Ser Pro Ser Ser Leu Ser Ala Ser Leu Gly
1               5                   10                  15

Asp Thr Ile Thr Ile Thr Cys His Ala Ser Gln Asn Ile Asn Val Trp
            20                  25                  30

Leu Ser Trp Tyr Gln Gln Lys Pro Gly Asp Ile Pro Lys Leu Leu Ile
        35                  40                  45

Tyr Lys Ala Ser Asn Leu His Thr Gly Val Pro Ser Arg Phe Ser Gly
    50                  55                  60

Ser Gly Ser Gly Thr Gly Phe Thr Leu Val Ile Ser Ser Leu Gln Pro
65                  70                  75                  80

Glu Asp Ile Ala Thr Tyr Tyr Cys Gln Gln Gly Arg Ser Tyr Pro Leu
                85                  90                  95

Thr Phe Gly Ala Gly Thr Lys Leu Glu Leu Lys
            100                 105

<210> 63
<211> 354
<212> DNA
<213> Mus musculus

<220>
<221> CDS
<222> (1)..(354)

<400> 63
gag gtg cag ctg ctc gag gag tct ggg gga ggc tta gtg aag cct gga      48
Glu Val Gln Leu Leu Glu Glu Ser Gly Gly Gly Leu Val Lys Pro Gly
1               5                   10                  15

ggg tcc ctg caa ctc tcc tgt tca gcc tct gga ttc act ttc agt agc      96
Gly Ser Leu Gln Leu Ser Cys Ser Ala Ser Gly Phe Thr Phe Ser Ser
            20                  25                  30

cat ttc atg tct tgg gtt cgc caa act cca gag aag agg ctg gag tgg      144
His Phe Met Ser Trp Val Arg Gln Thr Pro Glu Lys Arg Leu Glu Trp
        35                  40                  45

gtc gca tcc att agt agt ggt ggt gac agt ttc tat cca gac agt ctg      192
Val Ala Ser Ile Ser Ser Gly Gly Asp Ser Phe Tyr Pro Asp Ser Leu
    50                  55                  60

aag ggc cga ttc gcc atc tcc aga gat aat gcc agg aac atc ctg ttc      240
Lys Gly Arg Phe Ala Ile Ser Arg Asp Asn Ala Arg Asn Ile Leu Phe
65                  70                  75                  80

ctg caa atg agc agt ctg agg tct gag gac tcg gcc atg tat ttc tgt      288
Leu Gln Met Ser Ser Leu Arg Ser Glu Asp Ser Ala Met Tyr Phe Cys
                85                  90                  95

aca aga gac tac tct tgg tat gct ttg gac tac tgg ggt caa gga acc      336
Thr Arg Asp Tyr Ser Trp Tyr Ala Leu Asp Tyr Trp Gly Gln Gly Thr
            100                 105                 110

tca gtc acc gtc tcc tca                                              354
Ser Val Thr Val Ser Ser
        115

<210> 64
<211> 118
<212> PRT
<213> Mus musculus

<400> 64
Glu Val Gln Leu Leu Glu Glu Ser Gly Gly Gly Leu Val Lys Pro Gly
1               5                   10                  15

Gly Ser Leu Gln Leu Ser Cys Ser Ala Ser Gly Phe Thr Phe Ser Ser
            20                  25                  30

His Phe Met Ser Trp Val Arg Gln Thr Pro Glu Lys Arg Leu Glu Trp
        35                  40                  45

Val Ala Ser Ile Ser Ser Gly Gly Asp Ser Phe Tyr Pro Asp Ser Leu
    50                  55                  60

Lys Gly Arg Phe Ala Ile Ser Arg Asp Asn Ala Arg Asn Ile Leu Phe
65                  70                  75                  80

Leu Gln Met Ser Ser Leu Arg Ser Glu Asp Ser Ala Met Tyr Phe Cys
                85                  90                  95

Thr Arg Asp Tyr Ser Trp Tyr Ala Leu Asp Tyr Trp Gly Gln Gly Thr
            100                 105                 110

Ser Val Thr Val Ser Ser
        115

9

20

## Beschreibung

In der Beschreibung dieser Erfindung ist eine Anzahl von publizierten Dokumenten genannt. Der Gegenstand dieser Dokumente ist durch Bezugnahme in die Beschreibung inkorporiert.

Die Erfindung betrifft ein Verfahren zum Nachweis einer Infektion eines Säugers mit einem Säure-resistenten Mikroorganismus, wobei man (a) eine Stuhlprobe des Säugers mit mindestens zwei verschiedenen monoklonalen Antikörpern, Fragmenten oder Derivaten davon oder Aptameren unter Bedingungen inkubiert, die eine Komplexbildung von Antigenen aus dem Säure-resistenten Mikroorganismus mit den Antikörpern, Fragmenten oder Derivaten davon oder den Aptameren erlauben, und wobei (aa) der erste monoklonale Antikörper oder das Fragment oder Derivat davon oder das erste Aptamer ein Epitop des ersten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; (ab) der zweite monoklonale Antikörper oder das Fragment oder Derivat davon oder das zweite Aptamer ein vom Epitop des ersten Antigens verschiedenes Epitop eines zweiten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert, wobei die Teile der Säuger gemäß (aa) und gemäß (ab) überlappen können und in der Summe im wesentlichen die Gesamtanzahl der infizierten Säuger ausmachen; und (b) die Bildung mindestens eines Antigen-Antikörperkomplexes/Antigen-Aptamerkomlexes gemäß (aa) oder (ab) nachweist.

Vorzugsweise ist der Säure-resistente Mikroorganismus ein Bakterium, insbesondere *Helicobacter pylori, Helicobacter hepaticus* oder *Mycobacterium tuberculosis* oder *Campylobacter jejuni* oder *Campylobacter pylori.* Ferner bevorzugt ist, daß die beiden Epitope Epitope einer Urease und eines Hitzeschockproteins, vorzugsweise von Hsp60, einer Alkylhydroperoxid-Reduktase, vorzugsweise des 26kDa-Proteins, des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase) sind. In einer weiteren bevorzugten Ausführungsform der Erfindung wird die Stuhlprobe mit drei verschiedenen monoklonalen Antikörpern, Fragmenten oder Derivaten davon oder Aptameren untersucht, wobei der erste Antikörper, das Fragment oder Derivat davon oder das erste Aptamer ein Epitop einer Urease und der zweite Antikörper und der dritte Antikörper (oder das Derivat, Fragment oder Aptamer) je ein Epitop von unterschiedlichen vorstehend genannten Proteinen, vorzugsweise Hsp60 (wenn eines der beiden ersten Epitope ein Epitop einer Alkylhydroperoxid-Reduktase ist) oder eine Alkylhydroperoxid-Reduktase spezifisch bindet, wenn eines der beiden ersten Epitope ein Epitop von Hsp60 ist. Ferner betrifft die Erfindung diagnostische und pharmazeutische Zusammensetzungen und Testvorrichtungen, die die vorgenannten Komponenten enthalten sowie diese enthaltende Verpackungen.

Der Nachweis der Infektion eines Säugerörganismus mit einem mikrobiellen Pathogen oder Parasiten kann heute auf verschiedene, invasive wie nicht-invasive, direkte und indirekte Arten geführt werden. Beim Einsatz invasiver Techniken wird die körperliche Integrität des Untersuchten verletzt, z.B. bei der Entnahme einer Biopsie oder von Serum. Nicht-invasive Diagnosetechniken stellen Veränderungen in Parametern fest, die ohne einen Eingriff in den Organismus gemessen werden können. Bevorzugt werden hierzu Körperflüssigkeiten und Ausscheidungen wie Atemluft, Urin, Speichel, Schweiß oder Stuhl beprobt und analysiert. Bei direkten Methoden wird das Vorhandensein des Pathogens oder Parasiten, seiner Bestandteile oder deren Abbauprodukte nachgewiesen, z.B. durch Färbung und mikroskopische Untersuchung oder spezifische enzymatische Reaktionen. Indirekte Verfahren greifen zurück auf den Nachweis von Reaktionen des Wirtsorganismus auf das Pathogen oder den Parasiten, z.B. das Vorhandensein von Antikörpern gegen Antigene des Pathogens im Serum, im Urin oder im Speichel des Wirts. In einem weiteren indirekten Verfahren müssen dem Patienten Indikatorsubstanzen zugeführt werden, die von dem mikrobiellen Pathogen oder Parasiten in spezifischer Weise modifiziert werden, wobei die modifizierte Form in Proben nachgewiesen wird (Atemtest).

Ein Eingriff in den Organismus, z.B. zur Erlangung einer Gewebeprobe, ist für den Organismus in den meisten Fällen belastend und häufig auch mit hohem apparativen Aufwand und einem gesundheitlichen Risiko verbunden. Die vergleichsweise einfache Erfassung von Proben der oben beschriebenen Körperflüssigkeiten und Ausscheidungen machen daher nicht-invasive Techniken zur Methode der Wahl. Da weiterhin nicht jeder Wirt in gleicher Weise auf ein bestimmtes Pathogen oder einen bestimmten Parasiten reagiert, und die Reaktion des Wirts mit Verzögerung einsetzen und zudem auch nach Entfernung des Pathogens oder Parasiten aus dem Organismus persistieren kann, sind direkte Verfahren stets vorzuziehen. Idealerweise wird also eine Diagnose durch den nichtinvasiven, direkten Nachweis des Pathogens oder Parasiten in Körperflüssigkeiten oder Ausscheidungen geführt.

Ein diagnostisches Verfahren sollte darüber hinaus auch auf weitere Gesichtspunkte hin optimiert sein: Hohe Reproduzierbarkeit, Sensitivität und Spezifität, garantierte Verfügbarkeit der zu verwendenden Materialien in konstanter Qualität, geringe Kosten bei Herstellung und Durchführung, und einfache Anwendung unabhängig von aufwendigen Apparaturen sind hier zu berücksichtigende Parameter.

Aus den oben genannten Gründen nehmen Verfahren basierend auf der hohen Selektivität und Bindungsaffinität bestimmter Substanzklassen (z.B. Antikörper, Rezeptoren, Lektine, Aptamere) für molekulare Strukturen, welche so gewählt werden können, daß sie für den jeweils zu bestimmenden Stoff/Mikroorganismus hochspezifisch sind, in der medizinischen Diagnostik einen zunehmend breiten Raum ein. Insbesondere die Möglichkeit der Immobilisierung dieser Substanzen an Festkörperoberflächen sowie der Kopplung mit radioaktiven Nukliden, mit Enzymen, die in Kombination mit geeigneten Substraten Farbreaktionen auslösen, oder mit farbigen Partikeln mit der hochspezifischen Bindungsaffinität (z.B. ELISA = Enzyme linked immunosorbent assay) führte zu der Entwicklung kostengünstiger, einfacher und wenig zeitaufwendiger Nachweisverfahren für körpereigene wie körperfremde Stoffe.

In den Anfangsphasen der Entwicklung dieser Nachweisverfahren fanden ausschließlich polyklonale Antikörper Verwendung. Diese haben jedoch einige, dem Fachmann wohlbekannte Nachteile, so vor allem begrenzte Verfügbarkeit und oft auch Kreuzreaktivitäten. Die Erarbeitung von Verfahren zur Herstellung monoklonaler Antikörper (Köhler & Milstein (1975)), die Fortschritte bei der Isolierung von Rezeptoren und deren gerichtete Expression in zellulären Wirtssystemen, die Entwicklung von Lektinen mit hoher Affinität für bestimmte Kohlenhydrate sowie die Entdeckung, daß Nukleinsäuren (Aptamere) molekulare Strukturen spezifisch binden können, konnten diese Nachteile großenteils beseitigen. Heute lassen sich mit vergleichsweise einfachen Methoden die Spezifität und Sensitivität von Nachweisverfahren optimieren.

Aufgrund der hohen Spezifität eignen sich derartige Verfahren besonders zum Nachweis einzelner, definierter Substanzen wie Haptene, Peptide oder Proteine, vorausgesetzt, das erkannte Strukturelement ist konstant innerhalb der zu untersuchenden Probenpopulation und spezifisch für die nachzuweisende Substanz. Sie sind zudem für eine Messung in Körperflüssigkeiten gut geeignet, und stellen damit eine naheliegende Option für den direkten Nachweis von Pathogenen in dieser Probenmatrix dar. Entsprechend sind im Stand der Technik Verfahren zur Diagnose z.B. von *Entamoeba histolytica* (Haque (1993), J. Infect. Dis. 167: 247-9), enterohemorrhagischen *Escherichia coli* (EHEC; Park (1996), J. Clin. Microbiol. 34: 988-990), *Vibrio cholerae* (Hasan (1994), FEMS Microbiol. Lett. 120: 143-148), Torovirus-ähnlichen Partikeln (Koopmans (1993), J. Clin. Microbiol. 31: 2738-2744) oder *Taenia saginata* (Machnicka (1996), Appl. Parasitol. 37: 106-110) aus Stuhl beschrieben. Ein Diagnoseverfahren für Adenovirus-bedingte Gastroenteritis (Hermann (1987), J. Infect. Dis. 155: 1167-1171) verwendet die Kombination aus zwei, gegen unterschiedliche enterische Adenovirus-Serotypen gerichteten, monoklonalen Antikörpern, um diese Serotypen unbeeinflußt von der Präsenz anderer, nicht enterischer Adenoviren nachweisen zu können. Hierbei gelingt der Nachweis bei Kenntnis des Adenovirus-Serotyps, mit dem ein Patient identifiziert ist, auch mit dem jeweiligen monoklonalen Antikörper allein zuverlässig.

Den oben beschriebenen Pathogenen ist gemeinsam, daß sie im Darm, in der Darmschleimhaut oder im Darmlumen ihres Wirts, in allen Fällen des Menschen, lebens- und vermehrungsfähig sind. Sie besitzen also Mechanismen, die Ihnen das Überleben und die Vermehrung in Anwesenheit der im Darm aktiven Abbau- und Verdauungssysteme erlauben. Damit ist es wahrscheinlich, daß bei der Ausscheidung mit dem Stuhl eine hohe Anzahl intakter oder fast intakter Pathogene bzw. Parasiten abgehen. Mit Nachweisreagenzien, beispielsweise Antikörpern, welche Bestandteile der Pathogene bzw. Parasiten erkennen, können diese im Stuhl oder in aufbereiteten Stuhlproben in der Regel leicht nachgewiesen werden.

Es gibt jedoch eine Anzahl von Pathogenen und Parasiten, die einerseits aufgrund der Beziehungen der von ihnen befallenen Gewebe (z.B. Lunge, Magen, Pankreas, Duodenum, Leber) zum Magen-Darm-Trakt im Stuhl auftreten können, andererseits aber im Darm selbst nicht lebens- und/oder vermehrungsfähig sind. Diese Pathogene und Parasiten werden hier als Säure-resistente Mikroorganismen bezeichnet. Zu diesen Pathogenen und Parasiten gehören beispielsweise *Helicobacter pylori (H. pylori)* und *Helicobacter hepaticus, Mycobacterium tuberculosis* und andere Mycobakterien, *Chlamydia pneumoniae, Legionella pneumophilae, Pneumocystis carinii, Campylobacter jejuni, Campylobacter pylori* und andere. Einige dieser Erreger können z.B. im Sputum nachgewiesen werden, jedoch ist beispielsweise der Nachweis von *Mycobacterium tuberculosis* im Sputum nur während eines kurzen Zeitraums möglich, und zwar nachdem sich eine den Erreger enthaltende Kaverne geöffnet hat. Ein Nachweis wird ferner dadurch erschwert, daß es nicht immer möglich ist, von einem zu Untersuchenden eine Sputumprobe zu erhalten. Dies trifft z.B bei Kleinkindern, verwirrten Patienten oder Tieren zu. Andere Pathogene wie beispielsweise *Legionella pneumophilae* lassen sich anhand von Antigenen, die über die Niere in den Urin gelangen spezifisch nachweisen. Dies gelingt jedoch nur, wenn die im Urin befindliche Menge für den Nachweis ausreicht. Der Nachweis im Stuhl wäre hierzu eine begrüßenswerte Alternative.. Die Darmpassage ist bei diesen Organismen jedoch mit einem starken Angriff durch die Verdauungs- und Abbaumechanismen des Säugers und insbesondere der Darmflora. verbunden. Für den betrachteten Erreger spezifische molekulare Strukturen können dabei zerstört oder in ihrer Konzentration stark herabgesetzt werden.

Die Degradation der Erreger im Darm hat sich bei Säure-resistenten Bakterien als Problem für einen sicheren Nachweis in Stuhlproben erwiesen. Die Zahl der Keime, die in den Magen eines Infizierten gelangen, ist im Vergleich zu anderen, sich im Darm ansiedelnden Bakterien gering. Zudem müssen Keime und Keimbruchstücke nach Verlassen des Magens einen langen Weg durch den an Proteasen reichen Darm zurücklegen. Diese Umstände haben zur Folge, daß sich nur geringe Mengen intakter Proteine im Stuhl wiederfinden lassen. Dies bedingt auch, daß die für einen ELISA-Test nötige Kombination zweier Epitope auf einem Antigen nicht mehr notwendigerweise wie im nativen Protein gegeben ist, und nah nebeneinander liegende Epitope die größte Wahrscheinlichkeit haben, in einem Nachweis, der zwei Epitope auf demselben Molekül benötigt, ein positives Ergebnis zu zeigen. Die individuell unterschiedliche Verteilung von im Stuhl von Infizierten nachgewiesenen Antigenen weist zusätzlich auf individuelle Eigenschaften in der Prozessierung der Antigene beim Darmdurchgang hin. Ein erster Ansatz, dieses Problem zu verringern, wurde durch den Offenbarungsgehalt der EP-A 0 806 667 bereitgestellt. In dieser Anmeldung wurde gezeigt, daß polyklonale Antikörper mit dem Lysat eines bestimmten *H*. *pylori*-Stammes induziert werden konnten, die eine breitere Variabilität von Stämmen aus verschiedenen geographischen Regionen erkennen. Allerdings geht aus dieser Anmeldung nicht hervor, welche Antigene durch das Serum erkannt werden. Ferner wurde eine entsprechende Diagnose vermittels monoklonaler Antikörper ausgeschlossen. Angesichts der Tatsache, daß Immunseren trotz aller Standardisierungsbemühungen schwanken können, muß das in der o.g. Anmeldung entwickelte Verfahren für eine breite Anwendung als suboptimal betrachtet werden. Hinzu kommt, daß für die Bereitstellung der polyklonalen Seren immer wieder neue Tiere immunisiert werden müssen. Die entsprechenden Verfahren sind sowohl zeit- als auch kostenintensiv.

Aufgabe der vorliegenden Erfindung war somit, die vorgenannten nachteiligen Verfahren aus dem Stand der Technik zu verbessern und ein leicht standardisierbares sowie kostengünstiges Verfahren zum sicheren Nachweis von Säure-resistenten Mikroorganismen bereitzustellen.

Diese Aufgabe wird durch die in den Ansprüchen gekennzeichneten Ausführungsformen gelöst.

Somit betrifft die Erfindung ein Verfahren zum Nachweis einer Infektion eines Säugers mit einem Säure-resistenten Mikroorganismus, wobei man
(a) eine Stuhlprobe des Säugers mit mindestens zwei verschiedenen monoklonalen Antikörpern, Fragmenten oder Derivaten davon oder Aptameren unter Bedingungen inkubiert, die eine Komplexbildung von Antigenen aus dem Säure-resistenten Mikroorganismus mit den Antikörpern, Fragmenten oder Derivaten davon oder den Aptameren erlauben, und wobei
   (aa) der erste monoklonale Antikörper oder das Fragment oder Derivat davon oder das erste Aptamer ein Epitop des ersten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert;
   (ab) der zweite monoklonale Antikörper oder das Fragment oder Derivat davon oder das zweite Aptamer ein vom Epitop des ersten Antigens verschiedenes Epitop eines zweiten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert,
   wobei die Teile der Säuger gemäß (aa) und gemäß (ab) überlappen können und in der Summe im wesentlichen die Gesamtanzahl der infizierten Säuger ausmachen; und
(b) die Bildung mindestens eines Antigen-Antikörperkomplexes oder Antigen-Aptamerkomplexes gemäß (aa) oder (ab) nachweist.

Der Begriff "Säure-resistenter Mikroorganismus" im Sinne dieser Erfindung umfaßt jeden Mikroorganismus, der aufgrund seiner Eigenschaften/Anpassungsmechanismen an den Wirt den physikalischen und chemischen Einflüssen des Verdauungstrakts widersteht, so daß er durch einen vorzugsweise immunologischen Nachweis oder unter Verwendung von Aptameren detektierbar ist. Beispiele für derartige Säure-resistente Mikroorganismen sind *Helicobacter pylori, Helicobacter hepaticum, Mycobacterium tuberculosis, Mycobacterium pseudotuberculosis, Mycobacterium cansassii, Camplyobacter jejuni* und *Campylobacter pylori.*

Der Begriff "Stuhlprobe des Säugers" bedeutet im Sinne dieser Erfindung jede Stuhlprobe, die für das erfindungsgemäße Nachweisverfahren eingesetzt werden kann. Insbesondere fallen darunter Stuhlproben, die nach an sich bekannten Verfahren für diagnostische Tests aufbereitet worden sind. Die Aufbereitung erfolgt beispielsweise gemäß RIDASCREEN® Entamoeba Enzymimmunoassay (R-Biopharm GmbH, Darmstadt).

"Fragmente" oder "Derivate" von monoklonalen Antikörpern weisen im Sinne dieser Erfindung dieselbe Bindungsspezifität wie die monoklonalen Antikörper auf. Derartige Fragmente oder Derivate können nach üblichen Verfahren hergestellt werden; vgl. z.B. Harlow und Lane "Antibodies, A Laboratory Manual", CSH Press, Cold Spring Harbor, USA, 1988. Beispiele für Fragmente sind Fab-, F(ab')₂ oder Fv-Fragmente. Beispiele für Derivate sind scFv-Fragmente. Derivate können auch chemisch hergestellte Substanzen sein, die dieselben oder verbesserte Bindungseigenschaften wie die Antikörper aufweisen. Solche Substanzen können beispielsweise durch Peptidomimetics oder durch verschiedene Runden von Phage Display und nachfolgende Selektion auf verbesserte Bindungseigenschaften hergestellt werden. Unter Aptameren werden erfindungsgemäß Nukleinsäuren wie RNA, ssDNA (ss = Einzelstrang), modifizierte RNA oder modifizierte ssDNA verstanden, die eine große Vielzahl von Zielsequenzen mit hoher Spezifität und Affinität binden. Der Begriff "Aptamer" ist im Stand der Technik bekannt und definiert beispielsweise in Osborne et al., Curr. Opin. Chem. Biol. 1 (1997), 5-9, oder in Stull und Szoka, Pharm. Res. 12 (1995), 465-483.

Der Begriff "mindestens zwei" ist hinsichtlich der Anzahl von verwendeten Antikörpern etc. nach oben nicht limitiert und bedeutet beispielsweise drei, vier, fünf, sechs, sieben, acht, neun oder zehn Antikörper etc.

"Bedingungen, die eine Komplexbildung erlauben" sind vom Fachmann ohne weiteres einstellbar; s. auch Harlow und Lane, a.a.O., und sind beispielsweise physiologische Bedingungen.

Der Begriff "nach der Darmpassage eine Struktur aufweist, die der nativen Struktur entspricht" bedeutet im Sinne dieser Erfindung, daß das Epitop eines Antigens nach der Darmpassage von einem monoklonalen Antikörper, Derivat oder Fragment davon oder dem Aptamer erkannt wird, der/das gegen dasselbe Antigen/Epitop gewonnen wurde oder an dieses bindet, welches die Darmpassage nicht passiert hat. Mit anderen Worten, das Epitop/Antigen, das vom o.g. Antikörper bzw. Fragmenten oder Derivate davon oder Aptameren spezifisch gebunden wird, hat die Darmpassage hinsichtlich seiner Struktur unbeschadet oder im wesentlichen unbeschadet überstanden und ist nicht degradiert worden. Als Quelle für die native Struktur des Epitops/Antigens kann z.B. ein mit einer French-Press aufgeschlossener Bakterienextrakt, der mit üblichen Verfahren (vgl. z.B. Sambrook et al., "Molecular Cloning, A Laboratory Manual", 2. Auflage 1989, CSH Press, Cold Spring Harbor, USA) weiter aufgereinigt wurde, oder ein Bakterienlysat, das nach Standardverfahren weiter aufgereinigt wurde (z.B. Sambrook et al., a.a.O.), dienen.

Der Begriff "nach der Darmpassage eine Struktur aufweist, die der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert" bedeutet erfindungsgemäß, daß das vom monoklonalen Antikörper, Fragment, Derivat oder Aptamer erkannte Epitop einem Epitop entspricht, das vom Immunsystem eines Säugers, vorzugsweise eines Menschen, präsentiert wird. Die Mechanismen der Antigen-Präsentation sowie Mechanismen, die zur Prozessierung von Antigenen führen und die daraus resultierende Antikörpervielfalt sind im Stand der Technik bekannt und beispielsweise in Janeway und Travers, Immunologie, 2. Auflage 1997, Spektrum Akademischer Verlag GmbH, Heidelberg beschrieben. Diese Epitope können sich von den nativen Epitopen unterscheiden. Der Kontakt des Säugers mit den Mikroorganismen oder den Proteinen oder Fragmenten oder den synthetischen Peptiden kann durch natürliche Infektion (außer bei den synthetischen Peptiden) oder durch Immunisierung erfolgen. Für die Immunisierung können auch Extrakte, Lysate, synthetische Peptide etc. des Mikroorganismus/Proteins herangezogen werden. Geeignete Immunisierungsschemata sind im Stand der Technik bekannt und beispielsweise beschrieben in Harlow und Lane, a.a.O. Geeignete Antikörper können beispielsweise auch durch Immunisierung und/oder Screening auf Surrogate wie synthetische Peptide, rekombinant hergestellte Proteine, Extrakte, Lysate oder partiell verdaute Proteine gewonnen werden.

Der Begriff "spezifisch bindet" bedeutet erfindungsgemäß, daß der monoklonale Antikörper, das Fragment oder Derivat davon oder Aptamer keine oder im wesentlichen keine Reaktivität mit anderen Epitopen in Proben nicht infizierter Säuger aufweist.

Der Begriff "wobei die Teile der Säuger gemäß (aa) und gemäß (ab) überlappen können und in der Summe im wesentlichen die Gesamtanzahl der infizierten Säuger ausmachen" bedeutet, daß mindestens eines, gegebenenfalls aber auch zwei oder mehr Epitope/Antigene im Stuhl auftreten, die von den monoklonalen Antikörpern, Fragmenten oder Derivaten oder den Aptameren spezifisch gebunden werden und daß die monoklonalen Antikörper, Fragmente oder Derivate oder die Aptamere mindestens ein Epitop eines Antigens bei im wesentlichen jedem infizierten Säuger detektieren. Der Begriff "im wesentlichen die Gesamtzahl" bedeutet dabei, daß die Epitope und damit eine entsprechende Infektion mit dem Mikroorganismus bei mehr als 70%, vorzugsweise mindestens 75%, stärker bevorzugt mehr als 85%, besonders bevorzugt mehr als 90% und am meisten bevorzugt mehr als 95% der Betroffenen erfaßt werden kann. Idealerweise werden Infektionen bei 100% der Betroffenen nachgewiesen.

Der Begriff "Antigen-Antikörperkomplex" im Sinne dieser Erfindung umfaßt nicht nur Komplexe, die das Antigen mit dem nativen Antikörper eingeht, sondern auch solche, die es mit dessen Fragmenten oder Derivaten eingeht.

Überraschenderweise wurde erfindungsgemäß gefunden, daß mit einer limitierten Anzahl von monoklonalen Antikörpern, Fragmenten oder Derivaten davon oder Aptameren, die mindestens zwei verschiedene Epitope von verschiedenen Antigenen Säure-resistenter Mikroorganismen spezifisch binden, eine relativ sichere Diagnose der Infektion mit diesen Bakterien/Pathogenen durchgeführt werden kann. So können in dieser Ausführungsform der Erfindung Antigene einer aufbereiteten Stuhlprobe bespielsweise an eine Festphase gebunden werden und das infizierende Agens mit der limitierten Anzahl von in markierter Form vorliegenden monoklonalen Antikörpern, Fragmenten oder Derivaten davon oder Aptameren nachgewiesen werden. Sofern das nach der Darmpassage vorliegende Antigen (noch) in di- oder multimerer Form vorliegt, können die gleichen monoklonalen Antikörper, Fragmente oder Derivate davon oder Aptamere sowohl als Fänger wie auch als Detektor eingesetzt werden. Diese Ausführungsform umfaßt auch die Möglichkeit, daß ein Epitop mehrmals auf derselben Untereinheit vorkommt. Die Erfindung schließt Ausführungsformen mit ein, in denen weitere Epitope, die die vorgenannten Eigenschaften aufweisen, von weiteren monoklonalen Antikörpern oder Fragmenten oder Derivaten davon oder von weiteren Aptameren erkannt werden. Auch kann ein Antigen von verschiedenen monoklonalen Antikörpern oder Aptameren, die dasselbe oder unterschiedliche Epitope des Antigens erkennen, nachgewiesen werden. So können beispielsweise drei monoklonale Antikörper oder drei Aptamere drei unterschiedliche Epitope auf zwei Proteinfragmenten erkennen. Letztere Ausführungsformen sind dazu geeignet, die Sicherheit bei der Stellung der Diagnose noch weiter zu erhöhen. Im Falle höhergeordneter Strukturen, z.B. von Di- oder Multimeren, kann der gleiche monoklonale Antikörper auch mehrere Epitope im selben multimeren Protein im erfindungsgemäßen Verfahren binden. Beispiele derartiger Ausführungsformen werden nachfolgend genauer beschrieben.

Umfaßt von der Erfindung sind Ausführungsformen, bei denen nur monoklonale Antikörper oder Fragmente oder Derivate davon oder nur Aptamere eingesetzt werden, wie auch Ausführungsformen, bei denen in einem Test unterschiedliche Arten von Nachweisreagenzien eingesetzt werden. So ist es möglich, daß ein erster monoklonaler Antikörper mit einem zweiten Antikörperderivat oder ein erstes Aptamer mit einem zweiten Antikörperfragment eingesetzt wird, um nur zwei Beispiele zu nennen. Insofern bezeichnen die Begriffe "erste" und "zweite" das erste und zweite Nachweisreagens. Gemeint ist dabei nicht, daß immer zwei Antikörper, Derivate oder Fragmente davon oder immer zwei Aptamere eingesetzt werden.

Die erfindungsgemäßen Ergebnisse sind vor allem deshalb überraschend, da der Stand der Technik hiervon weggelehrt hatte. So wurde beispielsweise bei *H. pylori* gefunden, daß Hauptantigene in ELISA-Tests nicht die gewünschte Spezifität und Sensitivität aufweisen; vgl. Newell et al., Serodiag. Immunother. Infect. Dis. 3 (1989), 1-6. Darüber hinaus lehrt die EP-A 0 806 667, daß ein sicherer Nachweis von *H. pylori*-Infektionen mit monoklonalen Antikörpern aufgrund der genetischen Variabilität der *H. pylori*-Stämme nicht möglich sei.

Das erfindungsgemäße Verfahren ist gegenüber dem erwähnten Stand der Technik insbesondere deshalb von Vorteil, da mit lediglich zwei monoklonalen Antikörpern oder Fragmenten oder Derivaten davon oder Aptameren eine relativ sichere Diagnose ermöglicht wird. Vorzugsweise werden für den Nachweis, beispielsweise im ELISA Pärchen von Antikörpern, Fragmenten, Derivaten davon oder Aptameren eingesetzt, wobei die beiden Antikörper des Pärchens dasselbe oder unterschiedliche Epitope auf demselben Antigen binden. Beispielsweise bildet *H*. *pylori*-Urease multimere Strukturen aus mehreren gleichen wie auch unterschiedlichen Untereinheiten aus. Im ELISA oder anderen Assays können somit die gleichen Antikörper, Fragmente oder Derivate davon oder Aptamere als Fängerantikörper/aptamere wie auch als Detektionsantikörper/aptamere eingesetzt werden. Zum einen gewährt die Verwendung von monoklonalen Antikörpern, Fragmenten oder Derivaten davon oder von Aptameren einen leicht zu haltenden Standard bei der Zuverlässigkeit des Diagnoseverfahrens, was ein großer Vorteil im Vergleich zu bisher bekannten und für diesen Zweck eingeführten Diagnoseverfahren ist. Weiterhin entfällt das beispielsweise im Verfahren der EP-A 0 806 667 notwendige immer neue Immunisieren und nachfolgende Testen von Versuchstieren. Ein weiterer Vorteil des erfindungsgemäßen Verfahrens ist seine Ausgestaltung als direktes und nicht-invasives Verfahren, was die einleiteod genannten Annehmlichkeiten für den Patienten sowie die Zuverlässigkeit bei der Bestimmung des Krankheitsstadiums erhöht.

In einer bevorzugten Ausführungsform ist der Säure-resistente Mikroorganismus ein Säure-resistentes Bakterium.

Im Stand der Technik ist eine Reihe von Säure-resistenten Bakterien bekannt. In einer besonders bevorzugten Ausführungsform ist das Säure-resistente Bakterium ein Bakterium der Gattung Helicobacter oder der Gattung Mycobacterium oder der Gattung Campylobacter.

In einer anderen besonders bevorzugten Ausführungsform ist das Bakterium ein Bakterium der Spezies *Helicobacter pylori, Helicobacter hepaticum* oder ein Bakterium der Spezies *Mycobacterium tuberculosis* oder ein Bakterium der Spezies *Campylobacter jejuni* oder *Campylobacter pylori.*

In einer weiteren bevorzugten Ausführungsform ist das Epitop des ersten Antigens ein Epitop einer Urease, z.B. einer α-Urease oder einer β-Urease, und das Epitop des zweiten Antigens ein Epitop eines Hitzeschockproteins, einer Alkylhydroperoxid-Reduktase oder des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase).

Überraschenderweise wurde erfindungsgemäß gefunden, daß in einer Population von Säugern, insbesondere von menschlichen Patienten, deren Stuhl auf Infektionen mit Säure-resistenten Bakterien getestet wurde, im wesentlichen alle Mitglieder dieser Population mindestens eines der o.g. Epitope im Stuhl aufwiesen, so daß mit hoher Wahrscheinlichkeit mit einem Satz von nur zwei entsprechenden monoklonalen Antikörpern, Fragmenten oder Derivaten davon oder Aptameren eine relativ sichere Diagnose gestellt werden kann.

Besonders bevorzugt ist, daß die Urease die β-Urease von *H. pylori* ist.

In einer weiteren besonders bevorzugten Ausführungsform ist das Hitzeschockprotein ein Hsp60.

Weiterhin ist besonders bevorzugt, daß es sich bei dem Hsp60 um das Hsp 60 von *H. pylori* handelt.

In einer zusätzlich besonders bevorzugten Ausführungsform ist die Alkylhydroperoxid-Reduktase das 26kDa-Protein von *H. pylori.*

Auch ist bevorzugt, daß die weiteren vorstehend genannten Proteine Proteine von *H*. *pylori* sind.

In einer weiteren (bevorzugten) Ausführungsform umfaßt das erfindungsmäße Verfahren zum Nachweis einer Infektion eines Säugers mit einem säure-resistenten Mikroorganismus die folgenden Schritte, wobei man (a) eine Stuhlprobe des Säugers mit drei verschiedenen monoklonalen Antikörpern, Fragmenten oder Derivaten davon oder Aptameren unter Bedingungen inkubiert, die eine Komplexbildung von Antigenen aus dem Säure-resistenten Mikroorganismus mit den Antikörpern, Fragmenten oder Derivaten davon oder Aptameren erlauben, und wobei (aa) der erste monoklonale Antikörper oder das Fragment oder Derivat davon oder das erste Aptamer ein Epitop des ersten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; (ab) der zweite monoklonale Antikörper oder das Fragment oder Derivat davon oder das zweite Aptamer ein vom Epitop des ersten Antigens verschiedenes Epitop eines zweiten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; und (ac) der dritte monoklonale Antikörper oder das Fragment oder Derivat davon oder das dritte Aptamer ein vom Epitop des ersten und zweiten Antigens verschiedenes Epitop eines dritten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert, wobei die Teile der Säuger gemäß (aa), gemäß (ab) und gemäß (ac) überlappen können und in der Summe im wesentlichen die Gesamtanzahl der infizierten Säuger ausmachen, und die Bildung mindestens eines Antigen-Antikörperkomplexes/Antigen-Aptamerkomlexes gemäß (aa), (ab) oder (ac) nachweist.

Hinsichtlich der Definitionen und bevorzugten Ausführungsformen dieses Verfahrens wird auf die vorstehend erläuterten Ausführungsformen verwiesen. Die Erläuterungen zur Kombination verschiedenartiger Nachweisreagenzien beim Einsatz von mindestens zwei Nachweisreagenzien gelten hier entsprechend für drei Nachweisregenzien. So kann ein erster monoklonaler Antikörper mit einem zweiten Fragment und einem dritten Aptamer im Test kombiniert werden. Diese Kombination bedeutet selbstverständlich nicht, daß noch zwei weitere Aptamere im Test eingesetzt werden. Der Begriff "dritte" bezieht sich somit auf das dritte Nachweisreagens, unabhängig von dessen möglicher Struktur. Entsprechendes gilt für die Begriffe "erste" und "zweite".

Bevorzugt ist das Epitop des ersten Antigens ein Epitop einer Urease, vorzugsweise der β-Urease von *H. pylori,* das Epitop des zweiten Antigens ein Epitop eines Hitzeschockproteins, vorzugsweise von Hsp60, vorzugsweise aus *H. pylori,* und das Epitop des dritten Antigens ein Epitop einer Alkylhydroperoxid-Reduktase, vorzugsweise des 26kDa-Proteins von *H. pytori.* In anderen bevorzugten Ausführungsformen sind die Epitope des zweiten und des dritten Antigens Epitope des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase), in Kombination mit der Urease als erstem und ggf. dem Hsp60 oder dem 26kDa-Protein als zweiten oder dritten Antigen. In einer weiteren bevorzugten Ausführungsform sind die Epitope des zweiten Antigens Epitope von Hsp60 und die Epitope des dritten Antigens Epitope des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase). Weitere Ausführungsformen unter Nachweis von Kombinationen der vorstehend genannten Proteine bzw. deren Epitope, auch ohne Urease, sind möglich.

Bei Reihenuntersuchungen von Stuhlproben von mit *H. pylori* infizierten Patienten auf verschiedene *H. pylori*-spezifische Proteine (Urease, HSP60, Alkylhydroperoxid-Reduktase) mittels ELISA unter Verwendung monoklonaler Antikörper wurde gefunden, daß die Auffindbarkeit verschiedener Antigene in Stuhlproben verschiedener Patienten sehr inhomogen ist. Weiter fand sich überraschenderweise, daß Kombinationen aus monoklonalen Antikörpern, die teilweise überlappende Epitope erkennen, für den Nachweis dieser Antigene mittels Sandwich-ELISA eine sehr gute Eignung besaßen (siehe Tabelle 3).

Die monoklonalen Antikörper, Fragmente oder Derivate davon oder die Aptamere können erfindungsgemäß lineare oder Konformationsepitope erkennen und spezifisch binden. In einer weiteren bevorzugten Ausführungsform bindet mindestens einer der monoklonalen Antikörper bzw. eines der Fragmente, Derivate oder Aptamere ein Konformationsepitop.

In einer besonders bevorzugten Ausführungsform binden sämtliche monoklonalen Antikörper etc. Konformationsepitope.

Im Epitop-mapping wurden 5 der im Stuhl-ELISA besonders geeigneten murinen mAK (monoklonalen Antikörper) untersucht (Tabelle 3). Überraschenderweise konnte bei drei dieser Antikörper kein lineares Epitop bestimmt werden. Daher wurde angenommen, daß diese mAK Konformationsepitope erkennen. Diese Aussage wird für die anti-β-Urease Antikörper durch die Immunblotergebnisse bestätigt, die zeigen, daß die denaturierte Urease nicht mehr oder nur sehr schwach erkannt wird (siehe Tabelle 2).

Die Erfindung betrifft in einer anderen Ausführungsform ein Verfahren zum Nachweis einer Infektion mit *Helicobacter pylori* im Stuhl eines Säugers, wobei man (a) eine Stuhlprobe mit mindestens zwei verschiedenen monoklonalen Antikörpern, Fragmenten, Derivaten davon oder Aptameren unter Bedingungen inkubiert, die eine Antigen-Antikörper/Antigen-Aptamer-Komplexbildung erlauben, wobei (aa) der erste monoklonale Antikörper, das Fragment, Derivat davon oder das erste Aptamer spezifisch β-Urease oder ein Fragment davon bindet; (ab) der zweite monoklonale Antikörper, das Fragment, Derivat davon oder das zweite Aptamer spezifisch das 26kDa-Antigen oder ein Fragment davon bindet oder spezifisch Hsp60 oder ein Fragment davon bindet; und (b) die Bildung mindestens eines Antigen-Antikörperkomplexes/Antigen-Aptamerkomlexes gemäß (aa) oder (ab) nachweist.

Ferner betrifft die Erfindung ein Verfahren zum Nachweis einer Infektion mit *Helicobacter pylori* im Stuhl eines Säugers, wobei man (a) eine Stuhlprobe mit drei verschiedenen monoklonalen Antikörpern, Fragmenten, Derivaten davon oder Aptameren unter Bedingungen inkubiert, die eine Antigen-Antikörper/Antigen-Aptamer-Komplexbildung erlauben, wobei (aa) der erste monoklonale Antikörper, das Fragment, Derivat davon oder das erste Aptamer spezifisch β-Urease oder ein Fragment davon bindet; (ab) der zweite monoklonale Antikörper, das Fragment, Derivat davon oder das zweite Aptamer spezifisch Hsp60 oder ein Fragment davon bindet; und (ac) der dritte monoklonale Antikörper, das Fragment, Derivat davon oder das dritte Aptamer spezifisch das 26kDa-Antigen oder ein Fragment davon bindet; und (b) die Bildung mindestens eines Antingen-Antikörper/Antige-Aptamer-Komplexes gemäß (aa), (ab) oder (ac) nachweist.

Hinsichtlich der verwendeten Definitionen und anwendbaren bevorzugten Ausführungsformen wird auf die vorherigen Erläuterungen hingewiesen.

Die genannten Proteine in den beiden vorstehenden Ausführungsformen sind selbstverständlich *H. pylori*-Proteine.

In einer anderen bevorzugten Ausführungsform ist der Hsp60-spezifische Antikörper der von dem bei der Deutschen Sammlung von Mikroorganismen und Zellkulturen (DSMZ) am 23. Juni 1998 nach den Statuten des Budapester Vertrages unter der Hinterlegungsnummer DSM ACC2356 hinterlegten Hybridom HP16m/2A5-E6-E5 produzierte Antikörper.

In einer weiteren bevorzugten Ausführungsform ist der 26kDa-Antigen-spezifische Antikörper der von dem bei der Deutschen Sammlung von Mikroorganismen und Zellkulturen (DSMZ) am 23. Juni 1998 nach den Statuten des Budapester Vertrages unter der Hinterlegungsnummer DSM ACC2355 hinterlegten Hybridom HP15m/3E8-D9-D6 produzierte Antikörper.

In einer anderen bevorzugten Ausführungsform ist der β-Urease-spezifische Antikörper der von einem der bei der Deutschen Sammlung von Mikroorganismen und Zellkulturen (DSMZ) am 23. Juni 1998 nach den Statuten des Budapester Vertrages unter den Hinterlegungsnummern DSM ACC2360 oder DSM ACC2362 hinterlegten Hybridomen HP8m/4H5-D4-C9 oder HP9.1m/3C2-F8-E2 produzierte Antikörper. Der in den Beispielen/Figuren beschriebene β-Urease-spezifische Antikörper HP8m/1H5-G2-B4 wird durch einen Tochterklon des hinterlegten Hybridoms HP8m/4H5-D4-C9 produziert. Die beiden durch Mutter- und Tochterklon produzierten Antikörper werden durch identische DNA-Sequenzen kodiert und weisen dieselben Eigenschaften auf.

In einer besonders bevorzugten Ausführungsform weist die schwere Kette des ein Hsp60-Epitop bindenden Antikörpers mindestens eine der folgenden CDRs, vorzugsweise die CDR3 und weiter bevorzugt alle drei folgenden CDRs auf:
- CDR1:: GFSLSRYSVH
- CDR2:: MIWGGGSTDYNSGLKS
- CDR3:: NMGGRYPDYFDY

In einer weiteren besonders bevorzugten Ausführungsform weist die die schwere Kette des ein Hsp60-Epitop bindenden Antikörpers kodierende DNA-Sequenz mindestens eine der folgenden CDRs, vorzugsweise die CDR3 und weiter bevorzugt alle drei folgenden CDRs auf:
- CDR1:: GG GTTCTCATTA TCCAGATATA GTGTACAC
- CDR2:: ATGATATGGG GTGGTGGAAG CACAGACTAT AATTCAGGTC TCAAATCC
- CDR3:: AATATG GGGGGTAGGT ACCCGGACTA CTTTGACTAC

In einer anderen besonders bevorzugten Ausführungsform weist die leichte Kette des ein Hsp60-Epitop bindenden Antikörpers mindestens eine der folgenden CDRs, vorzugsweise die CDR3 und weiter bevorzugt alle drei folgenden CDRs auf
- CDR1:: RASKSVSTSGYSYIH
- CDR2:: LASNLES
- CDR3:: QHSRELPLT

Weiterhin weist in einer besonders bevorzugten Ausführungsform die die leichte Kette dieses Antikörpers kodierende DNA-Sequenz mindestens eine der folgenden CDRs, vorzugsweise die CDR3 und weiter bevorzugt alle drei folgenden CDRs auf:
- CDR1:: A GGGCCAGCAA GAGTGTCAGT ACATCTGGCT ATAGTTACAT ACAC
- CDR2:: C TTGCATCCAA CCTAGAATCT
- CDR3:: CAGC ACAGTAGGGA GCTTCCGCTC ACG.

In einer weiteren besonders bevorzugten Ausführungsform des erfindungsgemäßen Verfahrens weist die schwere Kette des ein 26kDa-Protein bindenden Antikörpers mindestens eine der folgenden CDRs, vorzugsweise die CDR3 und weiter bevorzugt alle drei folgenden CDRs auf:
- CDR1:: GFTFNSYAMY
- CDR2:: RIRSKSDNYATYYANSVKD
- CDR3:: DHDKFPFYYALDY

In einer anderen besonders bevorzugten Ausführungsform weist die die schwere Kette des ein Epitop des 26kDa-Proteins bindenden Antikörpers kodierende DNA-Sequenz mindestens eine der folgenden CDRs, vorzugsweise die CDR3 und weiter bevorzugt alle drei folgenden CDRs auf:
- CDR1:: GG TTTCACCTTC AATTCCTATG CCATGTAC
- CDR2:: CGCATAAGAA GTAAAAGTGA TAATTATGCA ACATATTATG CCAATTCAGT GAAAGAC
- CDR3:: GATCATG ATAAGTTTCC TTTTTACTAT GCTCTGGACT AC

In einer anderen besonders bevorzugten Ausführungsform des erfindungsgemäßen Verfahrens wird ein Antikörper, Fragment oder Derivat davon eingesetzt, dessen leichte Kette des ein 26kDa-Protein bindenden Antikörpers mindestens eine der folgenden CDRs, vorzugsweise die CDR3 und weiter bevorzugt alle drei folgenden CDRs aufweist:
- CDR1:: TASSSVSSSYLH
- CDR2:: STSNLAS
- CDR3:: HQYHRSPPT

Weiterhin weist die die leichte Kette dieses Antikörpers kodierende DNA-Sequenz in einer anderen besonders bevorzugten Ausführungsform mindestens eine der folgenden CDRs, vorzugsweise die CDR3 und weiter bevorzugt alle drei folgenden CDRs auf:
- CDR1:: A CTGCCAGCTC AAGTGTGAGT TCCAGTTACT TGCAC
- CDR2:: AGCACTTCCA ACCTGGCTTC T
- CDR3:: CAC CAGTATCATC GTTCCCCACC GACG

In einer anderen besonders bevorzugten Ausführungsform des erfindungsgemäßen Verfahrens weist die schwere Kette des ein Epitop der β-Urease bindenden Antikörpers mindestens eine der folgenden CDRs, vorzugsweise die CDR3 und weiter bevorzugt alle drei folgenden CDRs auf:
- CDR1:: GFTFSSHFMS
- CDR2:: SISSGGDSFYPDSLKG
- CDR3:: DYSWYALDY
oder:
- CDR1:: GYAFSTSWMN
- CDR2:: RIYPGDGDTNYNGKFKG
- CDR3:: EDAYYSNPYSLDY

In einer weiterhin besonders bevorzugten Ausführungsform weist die die schwere Kette des ein Epitop der β-Urease bindenden Antikörpers kodierende DNA-Sequenz mindestens eine der folgenden CDRs, vorzugsweise die CDR3 und weiter bevorzugt alle drei folgenden CDRs auf:
- CDR1:: GG CTACGCATTC AGTACCTCCT GGATGAAC
- CDR2:: CGGATTTATC CTGGAGATGG AGATACTAAC TACAATGGGA AGTTCAAGGG C
- CDR3:: GAG GATGCCTATT ATAGTAACCC CTATAGTTTG GACTAC
oder:
- CDR1:: GG ATTCACTTTC AGTAGCCATT TCATGTCT
- CDR2:: TCCATTAGTA GTGGTGGTGA CAGTTTCTAT CCAGACAGTC TGAAGGGC
- CDR3:: GACTAC TCTTGGTATG CTTTGGACTA C

In einer anderen besonders bevorzugten Ausführungsform des erfindungsgemäßen Verfahrens weist die leichte Kette des ein Epitop der β-Urease bindenden Antikörpers mindestens eine der folgenden CDRs, vorzugsweise die CDR3 und weiter bevorzugt alle drei folgenden CDRs auf:
- CDR1:: RASQSIGTRIH
- CDR2:: YGSESIS
- CDR3:: QQSNTWPLT
oder:
- CDR1:: HASQNINVWLS
- CDR2:: KASNLHT
- CDR3:: QQGRSYPLT

Des weiteren weist die die leichte Kette dieses Antikörpers kodierende DNA-Sequenz bevorzugt folgende CDRs auf:
- CDR1:: A GGGCCAGTCA GAGCATTGGC ACAAGAATAC AC
- CDR2:: TAT GGTTCTGAGT CTATCTCT
- CDR3:: CAACAA AGTAATACCT GGCCGCTCAC G
oder:
- CDR1:: C ATGCCAGTCA GAACATTAAT GTTTGGTTAA GC
- CDR2:: AAG GCTTCCAACT TGCACACA
- CDR3:: CAACAG GGTCGAAGTT ATCCTCTCAC G

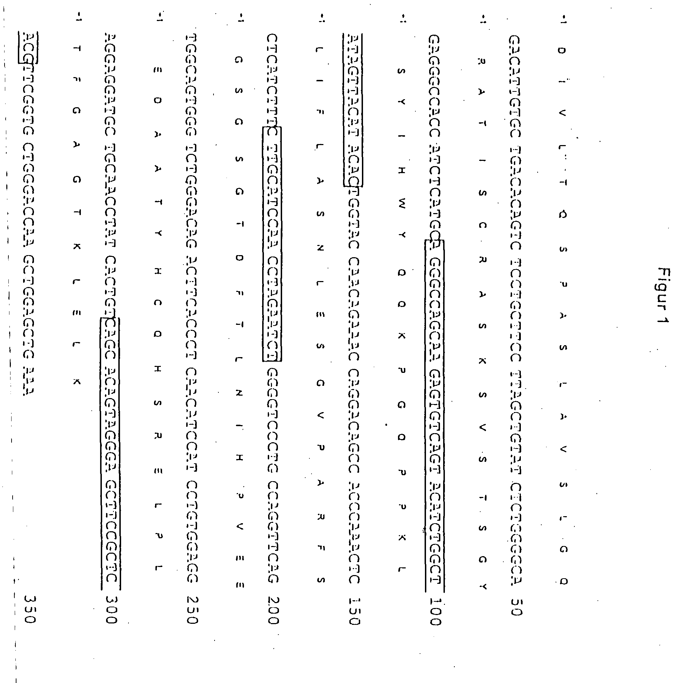

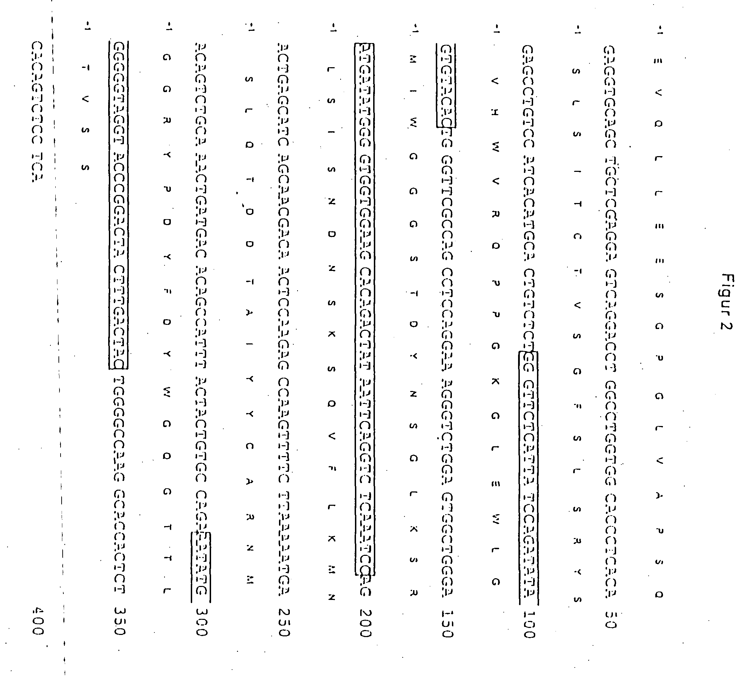

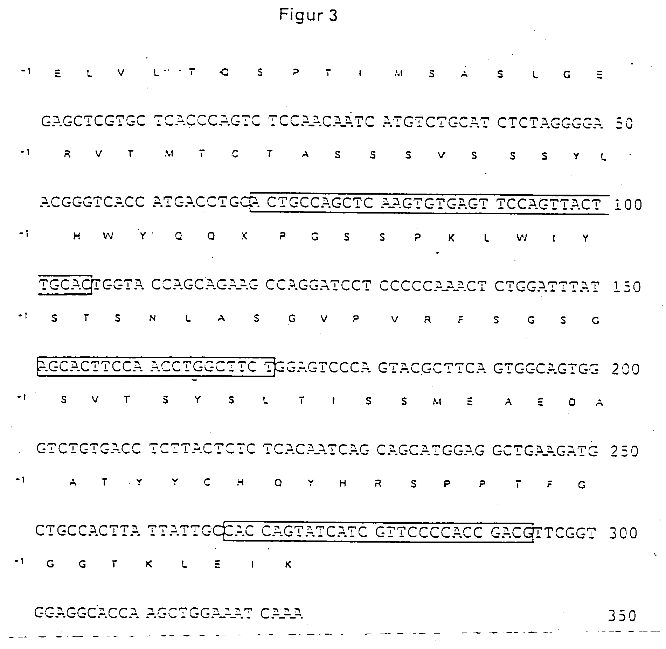

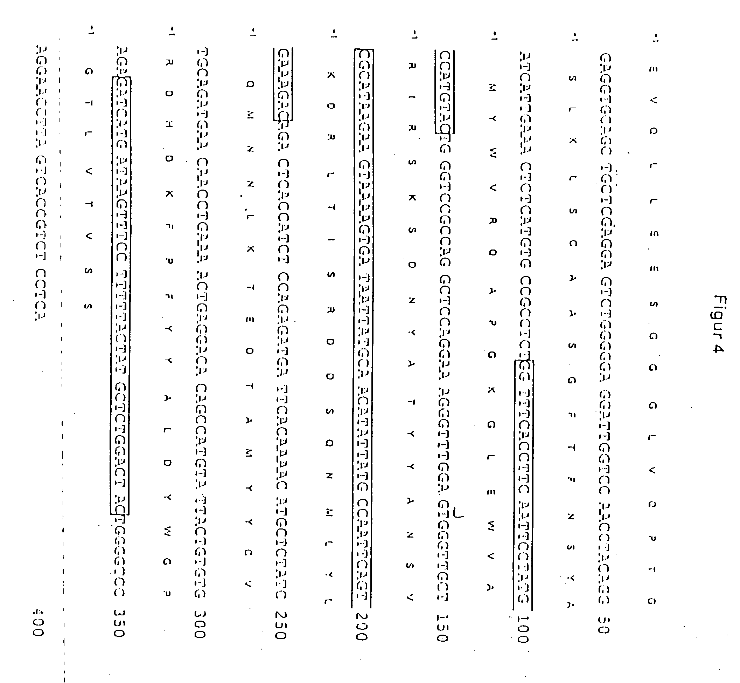

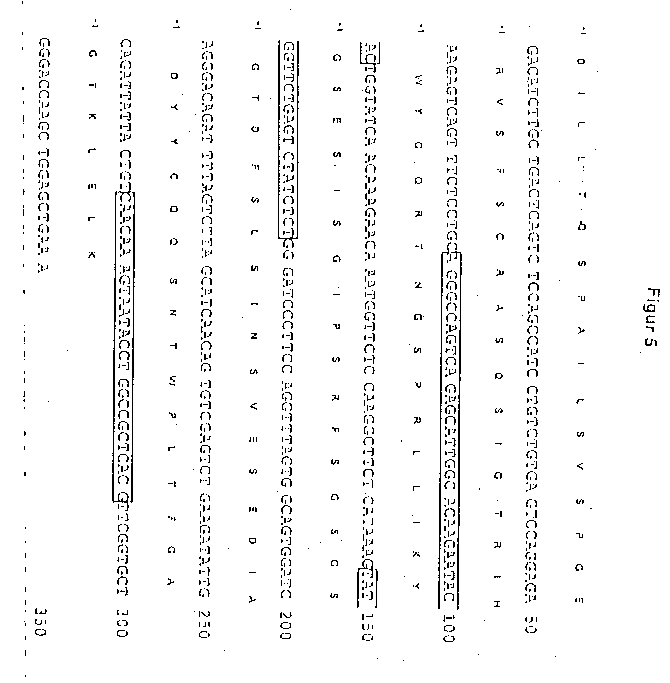

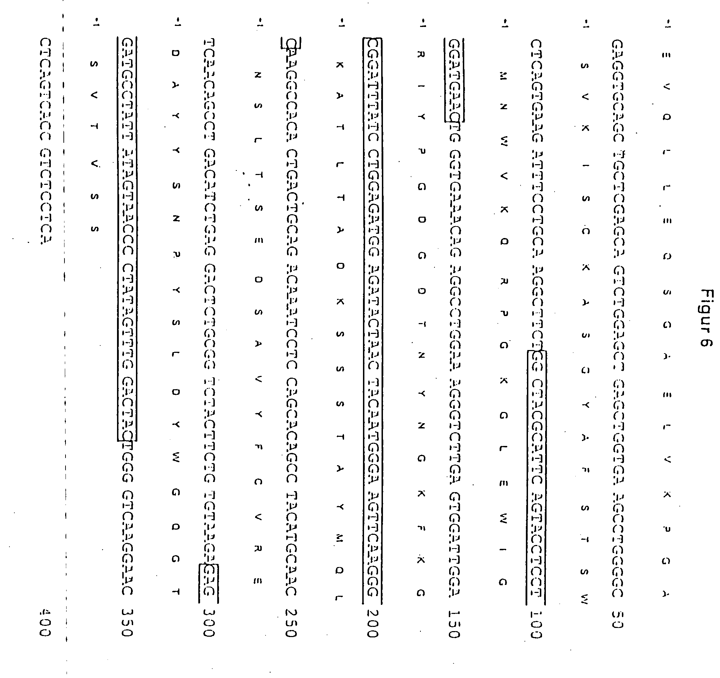

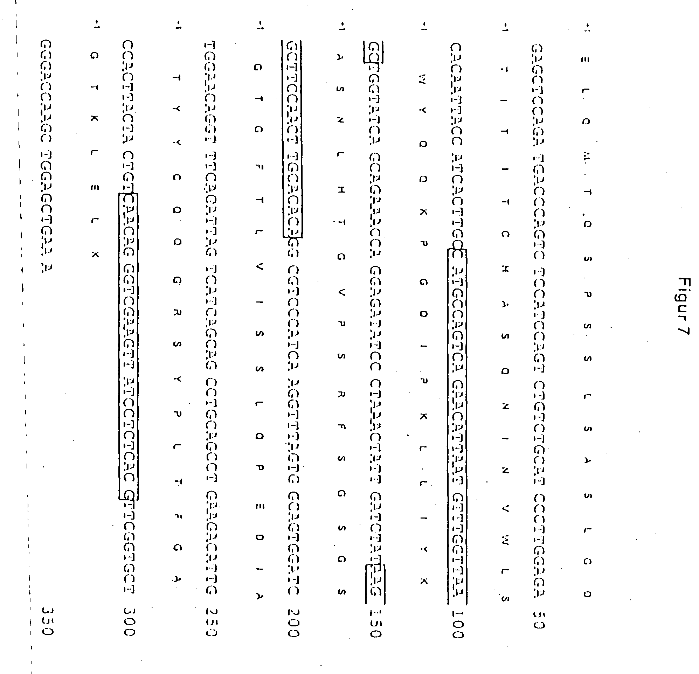

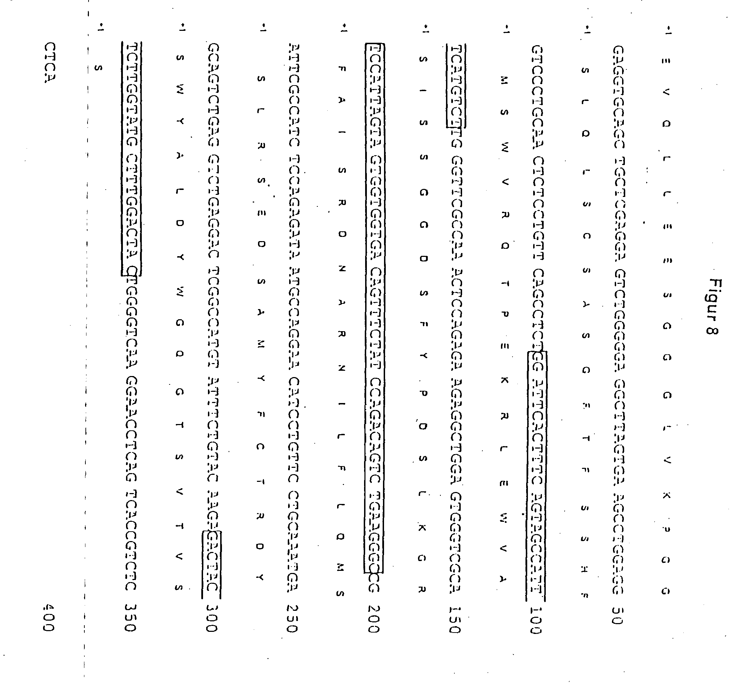

Besonders bevorzugt ist ferner, daß die schweren und leichten Ketten, die die vorstehend angegebenen CDRs aufweisen, gemeinsam in einem Antikörper, Fragment oder Derivat davon auftreten, der/das Hsp60, das 26kDa-Protein oder die β-Urease, oder ein Fragment davon, vorzugsweise aus *H. pylori* spezifisch bindet. Die Erfindung umfaßt jedoch auch Ausführungsformen, in denen diese schweren oder leichten Ketten mit anderen leichten bzw. schweren Ketten kombiniert werden, wobei die Bindungseigenschaften im wesentlichen beibehalten oder verbessert werden können. Entsprechende Verfahren sind im Stand der Technik bekannt. Besonders bevorzugte Antikörper weisen in den variablen Regionen der leichten und schweren Ketten die in den Figuren 1 und 2, den Figuren 3 und 4, den Figuren 5 und 6 oder den Figuren 7 und 8 dargestellten Aminosäuresequenzen auf bzw. werden die Regionen von den dort dargestellten DNA-Sequenzen kodiert.

In einer bevorzugten Ausführungsform werden mit der Stuhlprobe vor der Inkubation mit den Antikörpern folgende Schritte durchgeführt: Die Stuhlprobe wird 1:3 bis 1:25, vorzugsweise etwa 1:10 in einem Resuspendierungspuffer resuspendiert und daraufhin auf einem Vortexmixer gemischt. Ein Beispiel für einen Resuspendierungspuffer ist, 150 mM PBS, 0,1% SDS.

In einer weiteren bevorzugten Ausführungsform erfolgt der Nachweis der Bildung des mindestens einen Antigen-Antikörperkomplexes/Ahtigen-Aptamerkomplexes in Schritt (b) mittels eines immunologischen Verfahrens.

In einer anderen bevorzugten Ausführungsform erfolgt der Nachweis der Bildung des mindestens einen Antigen-Antikörperkomplexes/Antigen-Aptamerkomplexes in Schritt (b) mittels ELISA, RIA, Western Blot oder eines immunchromatographischen Verfahren.

Derartige Verfahren sind an sich im Stand der Technik bekannt; vgl. Harlow und Lane, a.a.O.

In einer besonders bevorzugten Ausführungsform des erfindungsgemäßen Verfahrens wird im immunologischen Verfahren, insbesondere im RIA oder im ELISA derselbe Antikörper oder dessen Fragment oder Derivat oder das Aptamer zur Bindung an die Festphase wie auch zum Nachweis des Epitops eingesetzt. Während der Fängerantikörper/das Fängeraptamer in unmodifizierter Form an die Festphase, beispielsweise eine Mikrotiterplatte, gebunden werden kann, ist der zur Detektion eingesetzte Antikörper, das Fragment oder Derivat davon bzw. das Aptamer gegebenenfalls mit einer Markierung versehen. Andererseits kann dieser Antikörper, das Fragment oder Derivat davon oder dieses Aptamer ebenfalls nicht markiert sein und damit das Epitop des Mikroorganismus, vorzugsweise das bakterielle Epitop auch über einen dritten markierten Antikörper, das Fragment oder Derivat davon ein drittes markiertes Aptamer nachgewiesen werden, wobei dieser Antikörper, das Fragment oder Derivat davon oder dieses Aptamer ein speziesspezifischer oder Ig-klassenspezifischer Antikörper oder ein entsprechendes Aptamer sein kann. Markierungen von Antikörpern, beispielsweise mit radioaktiven oder fluoreszierenden Markern sind im Stand der Technik bekannt; vgl. Harlow und Lane a.a.O. Entsprechendes gilt für Aptamere. Die vorstehend beschriebenen Ausführungsform ist besonders günstig zum Nachweis der Urease, vorzugsweise β-Urease, die ggf. auch nach der Darmpassage noch als Dimer, ggf. in multimerer Ausführung vorliegt. Selbstverständlich können auch in dieser Ausführungsform Kombinationen von Antikörpern, Fragmenten, Derivaten und Aptameren eingesetzt werden, z.B. Kombinationen von Antikörpern etc., die an unterschiedliche Epitope desselben Antigens binden.

In einer anderen bevorzugten Ausführungsform des erfindungsgemäßen Verfahrens ist der monoklonale Antikörper ein Maus-Antikörper.

Des weiteren sind in einer bevorzugten Ausführungsform die Antikörper, Fragmente oder Derivate davon oder die Aptamere an einen Träger fixiert.

Die Fixierung der Antikörper, Fragmente oder Derivate davon oder der Aptamere an einen Träger ist besonders vorteilhaft für die Durchführung von Routinechecks. Die Kombination Antikörper-Träger/Aptamer-Träger läßt sich ferner gut als Testbesteck oder in Kitform verpacken.

In einer besonders bevorzugten Ausführungsform ist das Trägermaterial ein poröses Trägermaterial.

In einer weiteren besonders bevorzugten Ausführungsform ist das Trägermaterial ein Teststreifen.

Zusätzlich besteht in einer bevorzugten Ausführungsform das Trägermaterial aus Zellulose oder einem Zellulosederivat.

Der Säuger, dessen Stuhl mit dem erfindungsgemäßen Verfahren untersucht werden kann, kann ein Tier, beispielsweise ein Haustier wie eine Katze oder ein Hund, ein Nutztier, z.B. ein Schwein oder ein sonstiges Tier wie eine Maus, ein Tiger oder ein Frettchen sein.

In einer bevorzugten Ausführungsform ist der Säuger ein Mensch.

Darüber hinaus betrifft die Erfindung einen monoklonaler Antikörper, ein Fragment oder Derivat davon, der/das eine V-Region aufweist, die eine Kombination der vorstehend dargestellten CDRs aufweist oder der von einem der vorstehend dargestellten Hybridomen produziert wird.

Bevorzugt ist dabei ein monoklonaler Antikörper, Fragment oder Derivat davon, der/das mindestens eine der in den Figuren 1 bis 8 dargestellten V-Regionen aufweist. Vorzugsweise weist dieser Antikörper zwei der in den Figuren 1 und 2, 3 und 4, 5 und 6 oder den Figuren 7 und 8 dargestellten V-Regionen auf. Auch ist bevorzugt, daß diese V-Regionen von den in den Figuren 1 bis 8 dargestellten DNA-Sequenzen kodiert werden.

In einer besonders bevorzugten Ausführungsform der Erfindung ist der monoklonale Antikörper, das Fragment oder Derivat davon ein Maus-Antikörper oder ein Fragment oder Derivat davon oder ein chimärer, vorzugsweise ein humanisierter Antikörper oder ein Fragment oder Derivat davon. Das Derivat kann auch ein Fusionsprotein sein. Weiter bevorzugt ist, daß der Antikörper markiert ist, beispielsweise mit einem Kolloid, mit einer radioaktiven, fluoreszierenden, phosphoreszierenden oder chemiluminiszierenden Markierung.
Die Herstellung von chimärisierten humanisierten und humanen Antikörpern und der anderen Derivate ist im Stand der Technik wohlbekannt (z.B. Vaughan et al.,1998; Orlandi et al., 1989, Harlow und Lane, a.a.O.).

Die Erfindung betrifft auch ein Aptamer, das dasselbe Epitop wie der monoklonale Antikörper, das Fragment oder Derivat davon spezifisch bindet. Die Herstellung derartiger Aptamere kann mit im Stand der Technik bekannten Verfahren erfolgen.

Weiterhin betrifft die Erfindung ein Epitop, das von einem der vorstehend beschriebenen monoklonalen Antikörper, Fragment oder Derivat davon oder Aptamer spezifisch gebunden wird.

Darüber hinaus betrifft die Erfindung weitere Antikörper, Derivate oder Fragmente davon, die das erfindungsgemäße Epitop spezifisch binden. Diese Antikörper können beispielsweise monoklonale Antikörper sein, die unter Verwendung des Epitops als Hapten/Bestandteil eines Antigens nach üblichen Verfahren hergestellt werden können.

Die vorliegende Erfindung betrifft darüber hinaus eine diagnostische Zusammensetzung enthaltend mindestens zwei monoklonale Antikörper, Fragmente oder Derivate davon oder Aptamere wie oben stehend definiert, gegebenenfalls fixiert an ein Trägermaterial.

Des weiteren betrifft die vorliegende Erfindung eine Testvorrichtung zum Nachweis mindestens eines wie oben stehend definierten Epitops, umfassend (a) mindestens zwei monoklonale Antikörper, Fragmente oder Derivate davon oder Aptamere wie obenstehend definiert, fixiert an ein Trägermaterial; (b) eine Vorrichtung zur Aufbereitung und Analyse von Stuhlproben und gegebenfalls (c) ein Gemisch von mindestens zwei monoklonalen Antikörpern, Fragmenten oder Derivaten davon oder Aptameren.

Die Erfindung hat ferner zum Gegenstand eine Testvorrichtung enthaltend (a) mindestens zwei monoklonale Antikörper, Fragmente oder Derivate davon oder Aptamere wie obenstehend definiert, wobei die Antikörper, Fragmente oder Derivate davon oder Aptamere konjugiert mit kolloidalem Gold, Latexpartikeln oder anderen farbgebenden Partikeln sind, deren Größe typischerweise im Bereich zwischen 5nm und 100nm, vorzugsweise zwischen 20nm und 60nm liegt; (b) eine Vorrichtung zur Aufbereitung und Analyse von Stuhlproben und gegebenfalls (c) ein Gemisch von mindestens zwei monoklonalen Antikörpern, Fragmenten oder Derivaten davon oder Aptameren.

Darüber hinaus betrifft die vorliegende Erfindung einen Kit enthaltend (a) mindestens zwei monoklonale Antikörper, Fragmente oder Derivate davon oder Aptamere wie obenstehend definiert, gegebenenfalls fixiert an ein Trägermaterial; gegebenenfalls (b) eine Vorrichtung zur Aufbereitung und Analyse von Stuhlproben und gegebenfalls (c) ein Gemisch von mindestens zwei monoklonalen Antikörpern, Fragmenten oder Derivaten davon oder Aptameren.

Die Erfindung betrifft auch eine Zusammensetzung enthaltend mindestens einen der vorstehend beschriebenen Antikörper oder eines der vorstehend beschriebenen Fragmente, Derivate oder Aptamere, gegebenenfalls in Kombination mit einem pharmazeutisch verträglichen Träger und/oder Verdünnungsmittel. Die Zusammensetzung ist vorzugsweise ein Arzneimittel.

Beispiele für geeignete pharmazeutisch verträgliche Träger sind dem Fachmann bekannt und umfassen Phosphat-gepufferte Kochsalzlösungen, Wasser, Emulsionen wie z.B. Öl/Wasser-Emulsionen, verschiedene Arten von Detergenzien, sterile Lösungen, etc. Arzneimittel, die solche Träger umfassen, können mittels bekannter konventioneller Methoden formuliert werden. Diese Arzneimittel können einem Individuum in einer geeigneten Dosis verabreicht werden, z.B. in einem Bereich von 1µg bis 100 mg pro Tag und Patient. Die Verabreichung kann auf verschiedenen Wegen erfolgen, z.B. intravenös, introperitoneal, subkutan, intramuskulär, lokal oder intradermal. Die Art der Dosierung wird vom behandelnden Arzt entsprechend den klinischen Faktoren bestimmt. Es ist dem Fachmann bekannt, daß die Art der Dosierung von verschiedenen Faktoren abhängig ist, wie z.B. der Größe, der Körperoberfläche, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden.

Schließlich betrifft die Erfindung eine Packung enthaltend die erfindungsgemäße diagnostische Zusammensetzung, die erfindungsgemäße Testvorrichtung oder den erfindungsgemäßen Kit.
Die Bestandteile der erfindungsgemäßen diagnostischen Zusammensetzung, der erfindungsgemäßen Testvorrichtung und/oder des erfindungsgemäßen Kits können in Behältern, wie beispielsweise Fläschchen oder Röhrchen, gegebenenfalls in Puffern und/oder Lösungen verpackt sein. Unter Umständen können eine oder mehrere der Bestandteile in ein- und demselben Behälter verpackt sein.

Die Figuren zeigen:
Fig. 1: DNA-Sequenz, die für eine leichte Kette eines für HSP60 spezifischen monoklonalen Antikörpers (DMS ACC2356) kodiert. Die DMSZ-Hinterlegungsnummer bezieht sich hier wie auch sonst in der Anmeldung auf das den monoklonalen Antikörper produzierende Hybridom. Die kodierte Aminosäuresequenz ist ebenfalls dargestellt.
Fig. 2: DNA-Sequenz, die für eine schwere Kette eines für HSP60 spezifischen monoklonalen Antikörpers (DMS ACC2356) kodiert. Die kodierte Aminosäuresequenz ist ebenfalls dargestellt.
Fig. 3: DNA-Sequenz, die für eine leichte Kette eines für 26kDa-Protein spezifischen monoklonalen Antikörpers (DMS ACC2355) kodiert. Die kodierte Aminosäuresequenz ist ebenfalls dargestellt.
Fig. 4: DNA-Sequenz, die für eine schwere Kette eines für 26kDa-Protein spezifischen monoklonalen Antikörpers (DMS ACC2355) kodiert. Die kodierte Aminosäuresequenz ist ebenfalls dargestellt.
Fig. 5: DNA-Sequenz, die für eine leichte Kette eines ersten für Urease spezifischen monoklonalen Antikörpers (DMS ACC2360) kodiert. Die kodierte Aminosäuresequenz ist ebenfalls dargestellt.
Fig. 6: DNA-Sequenz, die für eine schwere Kette eines ersten für Urease spezifischen monoklonalen Antikörpers (DMS ACC2360) kodiert. Die kodierte Aminosäuresequenz ist ebenfalls dargestellt.
Fig. 7: DNA-Sequenz, die für eine leichte Kette eines zweiten für Urease spezifischen monoklonalen Antikörpers (DMS ACC2362) kodiert. Die kodierte Aminosäuresequenz ist ebenfalls dargestellt.
Fig. 8: DNA-Sequenz, die für eine schweren Kette eines zweiten für Urease spezifischen monoklonalen DMS ACC2362) kodiert. Die kodierte Aminosäuresequenz ist ebenfalls dargestellt.

Die Beispiele erläutern die Erfindung.

### Beispiel 1: Isolierung von H. pylori-Antigenen

### Kultivierung von H. pylori

*H. pylori* (Stamm NCTC 11637) wurde in Petri-Schalen auf Wilkins-chalkern Agar unter Zusatz von 10% Pferdeblut sowie Amphotericin B, Vancomycin und Cefsoludin (Sigma Chemicals) ausgestrichen und 3-4 Tage in mikroaerophiler Atmosphäre (Anaerocult GasPAk, Merck) bei 37ºC inkubiert. Der Inhalt von 2 Schalen wurde in 350ml BHIB-Medium unter Zusatz der Antibiotika wie oben in einer 1l Flasche (Schott) suspendiert, das Medium für 10min mit einem Gasgemisch aus 10% CO₂, 5% O₂, 85% N₂ begast und die Flasche verschlossen. Die Kultur wurde für 2 Tage bei 37 ºC auf einem Rundschüttler geschüttelt, nach 24h wurde der Verschluß der Flasche geöffnet. Der Inhalt der Flasche wurde anschließend steril in eine 10l Flasche gegeben, mit 4,7l BHIB-Medium aufgefüllt, das Medium für 10min mit einem Gasgemisch aus 10% CO₂, 5% O₂, 85% N₂ begast und die Flasche verschlossen. Wiederum wurde für 2 Tage bei 37ºC auf einem Rundschüttler geschüttelt, nach 24h wurde der Verschluß der Flasche geöffnet. Das gesamte Volumen wird daraufhin bei 11000g für 10 min zentrifugiert, Überstand dekantiert und das Bakterienpellet gewogen. Zur Lagerung wurde der Pellet in einer physiologischen Kochsalzlösung unter Zusatz von 15% Glycerin im Verhältnis 2:1 (Gew.:Vol.) resuspendiert und bei -80ºC eingefroren. Um die Identität der kultivierten Bakterien zu überprüfen wurde eine visuelle Inspektion der Bakterien sowie Tests auf Urease-, Oxidase und Katalase-Aktivität durchgeführt. Die Freiheit von Kontamination wurde durch Kultur von etwas Suspension, entnommen vor dem Einfrieren, bei 37ºC an Luft für 48h belegt.

### Beispiel 2: Allgemeine Vorgehensweise zur Auswahl geeigneter Antigene zur Detektion auf Mikroorganismen

Nach Aufzucht wird ein Lysat des Mikroorganismus hergestellt. Die Auftrennung des Lysats erfolgt mittels Gelfiltration. Anschließend werden diejenigen Proteine isoliert, die in dem Lysat in großer Menge vorliegen. Diese Proteine werden mittels Proteinsequenzierung und den Vergleich mit Sequenzen aus geeigneten Datenbanken identifiziert. Hierdurch können charakteristische bzw. spezifische Proteine für den jeweiligen Mikroorganismus ermittelt werden. Für diese ausgewählten Antigene wird dann ein geeignetes Reinigungsschema entwickelt.

### Beispiel 3: Präparation von H. pylori Urease, Alkylhydroperoxid-Reduktase und HSP60

Die Präparation der drei *H. pylori*-Antigene Urease, Alkylhydroperoxid-Reduktase und HSP60 entspricht einer Modifikation einer bekannten Methode (Eschweiler et al., 1993). Gefrorenes Bakterienpellet wurde im Verhältnis 1:2 (Gew.:Vol.) mit 10% Butanol versetzt, auf einem Über-Kopf-Mischer bis zum vollständigen Auftauen und ca. 15min weitergeschüttelt. Nach Sedimentation für 20min bei 20.000g, 4°C, wurde der Überstand dekantiert und das Pellet erneut für 30min mit 10% Butanol wie oben extrahiert. Nach erneuter Sedimentation analog wie oben wurden die Überstände vereinigt und das Pellet zur weiteren Verwendung eingefroren.

### Isolierung von Urease und Alkylhydroperoxid-Reduktase (als Oligomere)

Die vereinigten klaren Überstände wurden durch 0,8µm Filter filtriert und sofort auf Source Q 16/10-Säule (mit 20mM HEPES, pH7.0, equilibriert) überführt. Mit 20mM HEPES, pH7.0, 2M NaCl eluierte Fraktionen wurden unmittelbar mittels eines Schnelltests auf Urease-Aktivität überprüft (4ml 1M Harnstoff, 1ml 0,2M HEPES, pH7,0 sowie 80µl einer Lösung von 5g/l Phenolrot wurden vermischt und mit aqua dest. auf 20ml aufgefüllt. 100µl dieser Lösung wurden anschließend mit der gleichen Menge einer Fraktion in ein Reagenzglas gegeben. Der Farbumschlag von gelb nach rot zeigte die Anwesenheit von Urease an). Urease-positive Fraktionen wurden auf eine mit Sephacryl S200 50/100 (Pharmacia) gefüllte Chromatographiesäule gegeben und aufgetrennt (Puffer: 50mM Tris, 100mM NaCl, 0,05% NaN₃, pH 7,3). Die Proteinkonzentration im Auslauf wurde durch Messung der optischen Dichte bei 280nm überwacht. Die Fraktionen des ersten Protein-Peaks enthielten oligomere Urease (Molekulargewicht ca. 550-600 kDa), gefolgt von einem zweiten Peak, das der Alkylhydroperoxid-Reduktase (ebenfalls als Oligomer) zuzurechnen war. Überschneidende Fraktionen wurden verworfen, reine Fraktionen (Überprüfung durch Elektrophorese auf Polyacrylamid-Gel) vereinigt und im Vakuum (Speed-Vac) oder durch Ultrafiltration eingeengt. Alkylhydroperoxid-Reduktase-Fraktionen, die noch größere Mengen Urease enthielten, wurden nachgereinigt mit Hilfe einer β-Urease Affinitätssäule (Antikörper Hp8m/1H5-G2B4, Connex, auf CNBr-aktivierter Sepharose 4B-Matrix, Amersham Pharmacia, Kopplung nach Angaben des Herstellers).

### Isolierung von HSP60 (als Oligomer)

Das HSP60 Antigen konnte nicht in ausreichender Quantität mit der oben beschriebene Butanol-Extraktionsmethode gewonnen werden. Deshalb wurden die Bakterien mit Ultraschall aufgeschlossen. Frisches Bakterienpellet oder das nach der Extraktion mit 10% Butanol erhaltene Pellet wurde aufgetaut, im Verhältnis 1:10 in 150mM PBS, pH 7,5 resuspendiert und in einem Falcon-Röhrchen auf Eiswasser in einem Sonikator (Sonifire) auf Stufe 25-30% (Stufe 7) Ultraschall-behandelt (4x90s mit je 90s Pause). Nach Zentrifugation bei 20.000g für 40min wurde der Überstand auf eine mit Sephacryl S200 50/100 (Pharmacia) gefüllte Chromatographiesäule aufgetragen und aufgetrennt (Puffer: 50mM Tris, 100mM NaCl, 0,05% NaN₃, pH7,3). HSP60 koeluierte mit Urease, daher wurden Urease-positive (siehe oben) Fraktionen vereinigt und auf einer β-Urease Affinitätssäule (siehe oben) nachgereinigt.

### Beispiel 4: Herstellung polyklonaler und monoklonaler Antikörper (pAK; mAK) gegen H. pylori-spezifische Antigene

Polyklonale Antiseren wurden von pab Productions (Hebertshausen) nach Standardverfahren gegen wie oben gereinigte Antigene hergestellt. Die Herstellung monoklonaler Antikörper erfolgt nach Methoden, die dem Fachmann bekannt sind (Harlow & Lane, 1988; Peters & Baumgarten, 1990).

### Immunisierung

Als Immunogen eingesetzt wurden die gereinigten Proteine Urease, HSP60 und Alkylhydroperoxid-Reduktase sowie rekombinante α- und β-Urease (Austral Biologics). Pro Immunogen wurden 4-6 Mäuse (BALB/c x C57 Black, F1-Generation, 8-12 Wochen alt) immunisiert (Priming) und in Abständen von ca. vier Wochen 3-4x geboostet. Pro Injektion wurden 200-300µl Antigenlösungen (25-50µg Antigen/Tier) im Verhältnis 1:1 mit kompletten (Priming) bzw. inkompletten (Boost) Freundschen Adjuvans (Difco) emulgiert und 100µl/Maus intraperitoneal injiziert. Vor der Fusion wurde den Mäusen retroorbital Blut entommen und aus dem daraus gewonnenen Antiserum der Antikörpertiter mittels Enzym-linked Immunosorbent Assay (ELISA, siehe unten) bestimmt.

### Fusion und Fusionsscreening

2-3 Tage nach dem letzten Boost wurden die Milz-Zellen der immunisierten Mäuse mit den Myelomzellen P3x63Ag8.653 (ATCC CRL-1580; Kearney et al., 1979) im Verhältnis 5:1 mit Polyethylenglykol 4000 fusioniert. Die fusionierten Zellen wurden in Klonierungsmedium (RPMI 1640 Medium+ 20% FCS + 200U IL-6/ml) mit Hypoxanthin-Aminopterin-Thymidin-Supplement (100x Konzentrat, Sigma) suspendiert und mit einer Zelldichte von 2-6x10⁴ Zellen/Napf auf 96-Napf-Mikrotiterplatten in Kultur genommen (37°C, 6% CO₂ und 95% relativer Luftfeuchtigkeit).
Nach ca. 10 Tagen wurde der Kulturüberstand im ELISA (siehe unten) auf das Vorhandensein von Antikörpern der gewünschten Spezifität gescreent.

### Etablierung und Kultivierung der Hybridome

Klone, die antigenspezifische Antikörper produzieren, wurden zweimal nach dem Prinzip der Grenzverdünnung (Limiting Dilution) (Coller & Coller, 1983) in Klonierungsmedium mit Hypoxanthin-Thymidin-Supplement (100x Konzentrat, Sigma) rekloniert. Der monoklonale Endklon wurde anschließend an die Flachflaschenkultur in RPMI 1640 Medium mit 10%FCS adaptiert und für die Kryokonservierung von 5-10 Aliquots mit je 2-5x10⁶ Zellen und die Produktion von antikörperhaltigen Kulturüberständen expandiert.

### ELISA

Für die Titerbestimmung und den Test der Kulturüberstände auf antigenspezifische Antikörper wurde der direkte ELISA gewählt. Die Beschichtung der ELISA-Platten (MaxiSorb; Nunc) erfolgte ü.N. bei 5°C mit einer Antigensuspension (2-6 µg Antigen/ml Carbonatpuffer, 0,1M, pH9,5). Zur Blockade der noch freien Bindungsstellen wurden 200µl PBS mit 2% Magermilchpulver (Gew.:Vol.) pro Napf pipettiert und 1h bei Raumtemperatur inkubiert. Die Inkubation von 100µl Kulturüberstand erfolgte 1-2h bei Raumtemperatur. Die Detektion der gebundenen Antikörper erfolgt durch Zugabe eines mit Meerrettich-Peroxidase (POD) konjugierten Sekundärantikörpers (Kaninchen-anti-Maus IgG-POD, DAKO). Die POD setzt dann im nächsten Schritt das farblose Substrat Tetramethylbenzidin (TMB, Sigma) in ein blaues Produkt um. Nach 5 bis 10 Minuten, oder sobald auch die Negativkontrolle eine leichte Blaufärbung zeigte, wurde die Reaktion durch Zugabe von 1N Schwefelsäure (100 µl/Napf) gestoppt. Die Stärke der Farbreaktion wurde im ELISA-Reader (MWG Spektral) gemessen. Die Messung erfolgt bei 455 nm gegen die Referenzwellenlänge 620 nm. Zwischen den einzelnen Arbeitsschritten wurde die ELISA-Platte 2-3x mit 250µl PBS mit 0,025% Tween 20 (Vol.:Vol.) gewaschen.

### Ergebnisse

Insgesamt wurden 24 mAK gegen β-Urease, 6 mAK gegen α-Urease, 8 mAK gegen 26kDa-Protein sowie 10 mAK gegen HSP60 generiert. Aus diesem Antikörper-Repertoire wurden diejenigen mAK mit den niedrigsten Nachweisgrenzen für die jeweiligen Antigene ausgewählt.

### Beispiel 5: Reinigung von mAK aus Hybridomakulturüberständen und Herstellung von Konjugaten

Die Reinigung von mAK aus Hybridomakulturüberständen erfolgt mittels der Protein-G Affinitätschromatographie (modifiziert nach: Pharmacia Biotech, 1994).
Die Kulturüberstände wurden filtriert (0,8µm) und direkt über die Protein G Matrix geleitet. Gewaschen wurde mit Tris/HCl bis das Signal am Detektor zum Hintergrund zurückgekehrt war. Eluiert wurde mit 0,1M Glycin/HCl, pH 3,0, die Detektion der Proteinkonzentration erfolgte im Eluat über optische Dichte bei 280nm. Alle Fraktionen im Bereich des einzigen Elutionssignals können verwendet werden.

Eine mögliche Verunreinigung mit bovinen IgG aus dem Serumzusatz ist als unkritisch einzustufen, da die gefundenen Nachweisantikörper nicht mit Rinder-Ig kreuzreagieren.
Die Kopplung monoklonaler Antikörper an Biotin und POD erfolgte nach Literaturmethoden (Harlow & Lane, 1988).

### Beispiel 6: Charakterisierung der monoklonalen Antikörper

### Isotypisierung

Die murinen mAK wurden mit dem lsotyping Kit IsoStrip von Boehringer Mannheim (Mannheim) isotypisiert.

### Immunblot

Für die Immunblotanalyse wurden pro Gel 30-50µg gereinigtes Antigen bzw. 300µg *H. pylori*-Lysat in reduzierendem Probenpuffer (Laemmli, 1970) gekocht und auf ein 12%iges präparatives SDS-Poplyacrylamid-Minigel (8,6x7,7x0,1cm, Biometra) aufgetragen. Die elektrophoretische Auftrennung erfolgte bei 25-30 mA/Gel.

Die im Gel aufgetrennten Proteine (Antigene) wurden dann im Semidry-Blot-Verfahren auf einer Nitrozellulose-Membran immobilisiert.

Die Membran wurde 30min bei Raumtemperatur mit 2% Magermilchpulver in PBS geblockt und dreimal 5min mit TBS/Tween 20 (0,2%) gewaschen. Für den folgenden Inkubationsschritt wurde die Membran in die Accutran Cross-Blot-Screening-Einheit (Schleicher und Schüll) eingespannt, unter Verwendung einer Gitterplatte mit 34 Querkanälen. In jeden der entstandenen Querkanäle wurden 250µl TBS/Tween 20 vorgelegt und je 250µl der zu testenden Hybridomakulturüberstände zugegeben. Die Inkubation erfolgte 2h bei Raumtemperatur unter Agitation.

Nach dreimaligem Waschen (s.o.) wurde die Membran 1h mit dem PODkonjugierten Sekundärantikörper (Kaninchen-anti-Maus IgG-POD, DAKO) inkubiert. Die Membran wurde dreimal gewaschen und der Immunkomplex durch Zugabe der 3,3-Diaminobenzidine-Substratlösung (DAB, Sigma) visualisiert.

### Charakterisierung von Antikörper-Antigen-Wechselwirkungen mittels Epitop-Kartierung

Um die Antikörperbindungsstelle an einem Protein identifizieren zu können, wurden von den *H. pylori*-Proteinen α- und β-Urease, HSP60 und 26kDa-Protein überlappende Peptidsequenzen (jeweils zwei Aminosäuren gegeneinander verschoben) mit 12 Aminosäureresten an einer Festphase synthetisiert und kovalent an eine Celluloseazetatmembran gekoppelt (Jerini GmbH, Berlin). Diese Membranen wurden mit Hybridomakulturüberstand inkubiert und die gebundenen Antikörper anschließend im Semidry-Blot Verfahren auf Nitrocellulosemembranen geblottet. Die Membran wurde geblockt und die immobilisierten Antikörper wie unter 5.2 beschrieben mit einem POD-markierten Sekundärantikörper (Kaninchen-anti Maus IgG-POD, DAKO) detektiert.

### Bestimmung der Nachweisgrenze für H. pylori-Antigene im ELISA

Zwischen den einzelnen Arbeitsschritten wurde die ELISA-Platte 2-3x mit 300µl PBS mit 0,025% Tween (Waschpuffer) (Vol.:Vol.) gewaschen. Die Beschichtung der ELISA-Platten (MaxiSorb; Nunc) erfolgte 1h bei 37ºC mit 100 µl einer Lösung eines polyklonalen Kaninchen-anti-*H*. *pylori*-Antigen Antikörpers (pAK; ca. 10µg Antikörper/ml Carbonatpuffer, 0,1M, pH9,5). Zur Blockade der noch freien Bindungsstellen wurden 200µl 150mM PBS mit 2% Magermilchpulver (Gew.:Vol.) pro Napf pipettiert und 30min bei Raumtemperatur inkubiert. 50ng/ml gereinigte *H. pylori*-Antigene gelöst in 150mM PBS unter Zusatz von 0,1% Magermilchpulver wurden in 1:2 Schritten verdünnt. Puffer diente als Negativ-Kontrolle. Anschließend wurden 100µl 1:10 verdünnten Kulturüberstands des zu untersuchenden mAK gegen das gleiche Antigen zugegeben und für 30-60min bei RT inkubiert. Die Detektion des gebundenen Antikörpers erfolgte wie unter 3.4 beschrieben. Die niedrigste Konzentration, bei der noch eine Extinktion größer oder gleich dem zweifachen der Kontrolle detektiert wurde, wurde als Nachweisgrenze akzeptiert.

**Tabelle 1: Ergebnisse zur Charakterisierung der mAK**

| **Fusion/Klon** | **Ag-Spezifität** | **Isotyp** | **IB (Ag)** | **IB (HP-Lysat)** | **NWG (ng Ag/ml)** |
|---|---|---|---|---|---|
| HP8m/4H5-D4-C9 | β-Urease | IgG1, κ | +/- | +/- | 0,6 |
| HP9.1m/2D1-F6-G1 | | IgG1, κ | - | +/- | 0,3 |
| HP9.1m/3C2-F8-E2 | | IgG1, κ | +/- | +/- | 1,2 |
| HP16.1m/3G1-H2-D7 | | IgG2a, κ | + | + | 0,6 |
| HP15m/3E8-D9-D6 | Alkylhydro-peroxid-Reduktase | IgG1, κ | + | + | 0,6 |
| HP15m/3F5-D5-B6 | | IgG1, κ | + | + | 0,3 |
| HP16m/2A5-E6-E5 | HSP60 | IgG1, κ | + | + | 3 |
| HP18.1m/3F11-G11-H11 | | IgG1, κ | + | + | 3 |
| HP18.1m/4D9-B9-A2 | | IgG1, κ | + | + | 3 |

| | | | | | |
|---|---|---|---|---|---|
| Abkürzungen: Ag Antigen; IB Immunblot; NWG Nachweisgrenze | | | | | |

### Ergebnisse

Tabelle 1 faßt die Ergebnisse der Isotypisierung, der Immunblotanalysen und der Nachweisgrenzenbestimmung für die in Tabelle 1 aufgelisteten mAK zusammen.

**Tabelle 2: Ergebnisse der Epitop-Kartierung monoklonaler Antikörper gegen H. pylori-Antigene**

| **Ag-Spezifität** | **Fusion/Klon** | **Epitop (AS-Position)** | **AS-Sequenz** |
|---|---|---|---|
| β-Urease | HP8m/1H5-G2-B4 | negativ | |
| (große Untereinheit) | HP8m/4H5-D4-C9 | negativ | |
| | HP9m/2B12-G7- | 369-375 | VGEVITR |
| | B12 | | |
| α-Urease | HP9m/2E7-B8-G10 | negativ | |
| (kleine Untereinheit) | HP9m/1H7-C6-D5 | negativ | |
| Alkylhydroperoxid- | HP15m/3E8-D9-D6 | negativ | |
| Reduktase | HP15m/3F5-D5-B6 | negativ | |
| | HP15m/4H12-A4- | negativ | |
| | D5 | 143-149 | LPLGRNA |
| | HP15m/1H7-H3-B7 | | |
| HSP60 | HP16m/2A5-E6-E5 | negativ | |
| | HP16m/1D10-A8 | negativ | |

| | | | |
|---|---|---|---|
| Abkürzungen: Ag Antigen; AS Aminosäure; negativ: Epitop konnte nicht kartiert werden | | | |

Bei zwei anti-α-Urease mAK und zwei anti-HSP60 mAK konnte mit der Immunblot-Methode kein Epitop kartiert werden. Von drei gegen die β-Urease gerichteten und vier gegen die Alkylhydroperoxid-Reduktase gerichteten mAK konnte nur bei je einem der mAK das Epitop bestimmt werden (siehe Tabelle 2). Diese Methode schien daher nicht geeignet, eventuelle Überlappungen der Epitope von Antikörpern gleicher Spezifität vorauszusagen.

### Beispiel 7: Charakterisierung von Antikörper-Antigen-Wechselwirkungen mittels Oberflächenplasmonresonanz-Spektroskopie

Die Epitop-Überlappungsmessung mit der SPR-Spektroskopie liefert Informationen über die gleichzeitige Zugänglichkeit von Antikörper-Epitopen. Damit lassen sich geeignete Antikörper-Pärchen für die Entwicklung von ELISA und Schnelltest finden (Fägerstam LG et al., 1990; Malmqvist M., 1996).

### Durchführung der Oberflächenplasmonresonanz-Spektroskopie am Pharmacia BlAcore

Alle Schritte wurden auf einer Pharmacia Biacore Processing Unit CA 186 nach Standardprotokollen durchgeführt (NHS/EDC, BlAcore Methods Manual). Hierzu wurde zunächst ein polyklonaler Kaninchen-anti-Maus-Antikörper auf der Dextranmatrix des BIAcore CM5 Glasträgerchips immobilisiert. Durch Zugabe des ersten *H. pylori*-antigenspezifischen monoklonalen Antikörpers (1. mAK; 100µg/ml; 10µl) wurde dieser mit dem immobilisiertem Fängerantikörper zur Reaktion gebracht und die entsprechende Veränderung am Detektor gemessen (ΔRU₁; RU Resonance Units). Nach Zugabe von *H*. *pylori*-Antigen (45µg/ml; 5µl) reagierte dies mit dem 1. mAK. Anschließend wurde verbliebener freier Fängerantikörper mit einem unspezifischen Mausantikörper (1mg/ml; 10µl) sowie oligomere Epitope durch Hinzufügen weiteren 1. mAK (100µg/ml; 10µl) geblockt. Nach der nun folgenden Reaktion des zweiten *H. pylori*-antigenspezifischen Antikörpers (2. mAK; 100µg/ml; 10µl) mit dem gebundenem Antigen wurde erneut die Veränderung im Signal des Detektors bestimmt (ΔRU₂) und zu der Veränderung am Detektor nach Zugabe des 1. mAK ins prozentuale Verhältnis gesetzt (ΔRU₂/ΔRU₁). Ein ΔRU₂/ΔRU₁<10% deutete auf überlappende Epitope hin. (Als überlappend werden die Epitope eines Antigens dann bezeichnet, wenn ein erster Antikörper alle von ihm zu bindenden Epitope eines Antigens besetzt hat und die nachfolgende Bindung eines zweiten Antikörpers be- bzw. verhindert). Durch kinetische Messungen lassen sich weiterhin die Werte für Geschwindigkeitskonstanten der Adsorption und Desorption von Antikörpern errechnen.

### Ergebnisse

Es wurden zwölf monoklonale anti-Urease-Antikörper und fünf Antikörper gegen Alkylhydroperoxid-Reduktase getestet. Durch Vergleich der 144 bzw. 25 Kombinationsmöglichkeiten untereinander wurden diejenigen Kombinationen bestimmt, die eine gleichzeitige Zugänglichkeit der Epitope zeigten und deren Affinitätskonstanten für die Verwendung im Stuhl-ELISA günstig erschienen. Tabelle 3 zeigt eine Auswahl der Ergebnisse aus der Kombination verschiedener Antikörper gegen Urease und stellt die kinetischen Konstanten der Eignung dieser Antikörper-Kombinationen im Stuhl-ELISA (Siehe Beispiel 9) gegenüber. Die Eignung von Antikörperpaaren für den ELISA muß sich hierbei nicht auf unabhängige Epitope beschränken. Auch eine Kombination aus Antikörpern, die z.T. überlappende Epitope erkennen (wie beispielsweise HP9.1m-2D1 und HP8m-4H5), ist dadurch zu erklären, daß oligomere Proteine wie Urease ein gleiches Epitop mehrmals präsentieren. Dies ermöglicht im ELISA die Bindung eines Fang-Antikörpers an ein Epitop und die gleichzeitige Bindung eines oder mehrerer Detektions-Antikörper an ein oder mehrere gleiche Epitope des oligomeren Proteins.

**Tabelle 3: Vier mAK-Pärchen-Beziehungen für Urease bestimmt mittels Oberflächenplasmonenresonanz und Ihr Verhalten im ELISA**

| **Fänger** | **k_{off}/kₒₙ** | **Detektor** | **k_{off}/kₒₙ** | **Epitope** | **Bewertung im ELISA** |
|---|---|---|---|---|---|
| HP9.1m -2B11 | 5,9*10⁻⁴/7,3*10⁴ | HP9.1m -2D1 | 1,1*10⁻⁴/3,7*10⁴ | unabhängig | - |
| HP9.1m -2B11 | 5,9*10⁻⁴/7,3*10⁴ | HP9.1m -3G 1 | 4,9*10⁻⁴/7,6*10⁴ | z.T. überlappend | - |
| HP9.1m -2D1 | 1,1*10⁻⁴/3,7*10⁴ | HP8m -4H5 | 4,5*10⁻⁶/4,8*10⁴ | z.T. überlappend | +++ |
| HP9.1m -XG1 | 3,0*10⁻⁴/1,5*10⁵ | HP9.1m -3G1 | 4,9*10⁻⁴/7,6*10⁴ | unabhängig | ++ |
| HP8m -4H5 | 4,5*10⁻⁶/4,8*10⁴ | HP9.1m -3C2 | 2,6*10⁻⁴/7,4*10⁴ | unabhängig | +++ |

### Beispiel 8: Auswahl von Antikörperpaaren für die Verwendung im ELISA am menschlichen Stuhl

Bei denjenigen Antikörpern, welche die niedrigsten Nachweisgrenzen bei der Messung aus dem Kulturüberstand zeigten, wurden mittels Oberflächenplasmonresonanz Epitopüberlappungen bestimmt. Die Kombinationen, die sich bei diesen Messungen vielversprechend zeigten (möglichst geringe Epitopüberlappung, hohe Geschwindigkeitskonstante für Adsorption, niedrige Geschwindigkeitskonstante der Desorption) wurden auf ihre Nachweisgrenze im Stuhl-ELISA getestet.

### Beispiel 9: Detektion von H, pylori im menschlichen Stuhl mittels ELISA

Abkürzungen: Detektor: Detektions-Antikörper; Fänger: Fang-Antikörper

Als Testproben standen Stuhlproben von Patienten aus dem Klinikum Großhadern, München, zur Verfügung, deren Infektionsstatus mit *H. pylori* (Kategorien 0 und 4) mittels serologischer und histologischer Untersuchungen erhoben wurde. In die Kategorie 0 wurden Patienten eingeteilt, bei denen das klinische Ergebnis aus Serologie und Histologie eindeutig negativ war, bei Patienten der Kategorien 4 das klinische Ergebnis aus Serologie und Histologie eindeutig positiv war.
Zwischen den einzelnen Arbeitsschritten wurde die ELISA-Platte 2-3x mit 250µl PBS unter Zusatz von 0,025% (Waschpuffer 1) bzw. 0,2% Tween 20 (Waschpuffer2; Vol.:Vol.) gewaschen. Die Beschichtung der ELISA-Platten (MaxiSorb; Nunc) erfolgte für 1h bei 37°C mit 100 µl einer mAK-Lösung (2.5 µg Antikörper/ml Carbonatpuffer, 0,1M, pH9,5). Zur Blockade der noch freien Bindungsstellen wurden 200µl 150mM PBS mit 0,2% Fischgelatine oder 1% Magermilchpulver (Gew.:Vol.) pro Napf pipettiert und 30min bei Raumtemperatur inkubiert. Gewaschen wurde mit Waschpuffer 1. Humanstuhl wurde im Verhältnis 1:10 (Gew.:Vol.) mit 150mM PBS unter Zusatz von 2% Magermilchpulver suspendiert, gereinigte *H. pylori*-Antigene gelöst in 150mM PBS in bekannten Konzentrationen zugegeben. Je 100µl der Suspensionen wurden pro Napf für 1h inkubiert. Die Platte wurde zuerst von Hand gespült und 4x mit Waschpuffer 2 gewaschen. Anschließend wurden 100µl einer Lösung mit Biotin-gekoppelten mAK (0.5 µg Antikörper/ml PBS) gegen das gleiche Antigen zugegeben und für 30-60min bei RT inkubiert. Die Detektion der gebundenen Antigene erfolgt durch Zugabe eines Konjugats von Streptavidin mit POD (DAKO). Die POD setzt das farblose Substrat TMB (Sigma) in ein blaues Produkt um. Nach 5 bis 10 Minuten, oder sobald auch die Negativkontrolle eine leichte Blaufärbung zeigte, wurde die Reaktion durch Zugabe von 1N Schwefelsäure (100 µl/Napf) gestoppt. Die Stärke der Farbreaktion wurde im ELISA-Reader (MWG Spektral) gemessen. Die Messung erfolgt bei 455 nm gegen die Referenzwellenlänge 620 nm.

**Tabelle 4:** Nachweis von *H. pylori*-Antigenen aus dem Stuhl sicher negativer (Kategorie 0) bzw. sicher positiver (Kategorie 4) Patienten mittels ELISA unter Verwendung monoklonaler Antikörper:

**Tabelle 4a: Kategorie 0 Proben: 0 bedeutet falsch-positiv, 1 bedeutet richtig-negativ**

| | **[µg Ag/ g Stuhl]** | | | **resultierender Wahrheitswert** | | | |
|---|---|---|---|---|---|---|---|
| **Probe** | **Urease** | **26kDa** | **HSP60** | **Urease** | **26kDa** | **HSP60** | **3-Kombination** |
| **cut-off** | | | | **1** | **15** | **5** | |
| 0047 | 0,6 | 2,3 | | 1 | 1 | | 1 |
| 0048 | 0,6 | 2,3 | | 1 | 1 | | 1 |
| 0051 | 2,1 | 22 | | 0 | 0 | | 0 |
| 0057 | 0,6 | 2,3 | | 1 | 1 | | 1 |
| 0069 | 0,6 | 2,3 | | 1 | 1 | | 1 |
| 0074 | 0,6 | 2,3 | | 1 | 1 | | 1 |
| 0087 | 0,6 | 2,3 | | 1 | 1 | | 1 |
| 0099 | 0,6 | 2,3 | | 1 | 1 | | 1 |
| 0185 | 0,6 | 2,3 | | 1 | 1 | | 1 |
| 0186 | 0,6 | 2,3 | | 1 | 1 | | 1 |
| 0189 | 0,6 | 2,3 | | 1 | 1 | | 1 |
| 0265 | 0,6 | 2,3 | | 1 | 1 | | 1 |
| 0298 | 0,6 | 2,3 | | 1 | 1 | | 1 |
| 0305 | 0,6 | 2,3 | | 1 | 1 | | 1 |
| 0373 | 0,6 | 2,3 | | 1 | 1 | | 1 |
| 0074 | 0,6 | 2,3 | 2,3 | 1 | 1 | 1 | 1 |
| 0089 | 0,6 | 2,3 | 2,3 | 1 | 1 | 1 | 1 |
| 0090 | 0,6 | 2,3 | 2,3 | 1 | 1 | 1 | 1 |
| 0091 | 0,76 | 2,3 | 2,3 | 1 | 1 | 1 | 1 |
| 0097 | 1,6 | 2,3 | 2,3 | 0 | 1 | 1 | 0 |
| 0103 | 1 | 2,4 | 15 | 1 | 1 | 0 | 0 |
| 0107 | 1 | 12 | 2,3 | 1 | 1 | 1 | 1 |
| 0114 | 4 | 2,3 | 44 | 0 | 1 | 0 | 0 |
| 0126 | 0,6 | 12 | 2,3 | 1 | 1 | 1 | 1 |
| 0130 | 0,76 | 67 | 2,3 | 1 | 0 | 1 | 0 |
| 0141 | 20 | 220 | 105 | 0 | 0 | 0 | 0 |
| 0142 | 1,8 | 2,3 | 2,3 | 0 | 1 | 1 | 0 |
| 0144 | 0,6 | 2,3 | 2,3 | 1 | 1 | 1 | 1 |
| 0146 | 0,6 | 2,3 | 2,3 | 1 | 1 | 1 | 1 |
| 0148 | 0,6 | 2,3 | 2,3 | 1 | 1 | 1 | 1 |
| 0168 | 0,6 | 2,3 | 2,3 | 1 | 1 | 1 | 1 |
| 0193 | 0,6 | 2,3 | 20 | 1 | 1 | 0 | 0 |
| 0207 | 0,6 | 2,3 | 20 | 1 | 1 | 0 | 0 |
| 0220 | 0,6 | 2,3 | 2,2 | 1 | 1 | 1 | 1 |
| 0225 | 0,6 | 2,3 | 2,2 | 1 | 1 | 1 | 1 |
| 0231 | 0,6 | 2,3 | 2,2 | 1 | 1 | 1 | 1 |
| 0235 | 0,6 | 2,3 | 2,2 | 1 | 1 | 1 | 1 |
| 0251 | 0,6 | 2,3 | | 1 | 1 | | 1 |
| 0258 | 0,6 | 95 | | 1 | 0 | | 0 |
| 0274 | 0,6 | 2,3 | | 1 | 1 | | 1 |

**Tabelle 4b: Kategorie 4 Proben: 0 bedeutet falsch-negativ, 1 bedeutet richtig-positiv**

| | **[µg Ag/ g Stuhl]** | | | **resultierender Wahrheitswert** | | | |
|---|---|---|---|---|---|---|---|
| **Probe** | **Urease** | **26kDa** | **HSP60** | **Urease** | **26kDa** | **HSP60** | **3-Kombination** |
| **cut-off** | | | | **1** | **15** | **5** | |
| 0006 | 1,9 | 25 | 34 | 1 | 1 | 1 | 1 |
| 0010 | 5,6 | 91,5 | 170 | 1 | 1 | 1 | 1 |
| 22 | 7,8 | 45 | 25 | 1 | 1 | 1 | 1 |
| 0350 | 15,6 | 198 | 95 | 1 | 1 | 1 | 1 |
| 0288 | 18 | 34 | 65,9 | 1 | 1 | 1 | 1 |
| 0332 | 29 | 360 | 650 | 1 | 1 | 1 | 1 |
| 0218 | 7 | 24 | 3 | 1 | 1 | 0 | 1 |
| 0285 | 7,4 | 115 | 3 | 1 | 1 | 0 | 1 |
| 0004 | 9,5 | 2,3 | 350 | 1 | 0 | 1 | 1 |
| 11 | 21 | 15 | 26 | 1 | 0 | 1 | 1 |
| 8 | 105 | 2,3 | 89 | 1 | 0 | 1 | 1 |
| 0003 | 1,5 | 2,3 | 10 | 1 | 0 | 1 | 1 |
| 15 | 2,3 | 2,3 | 15 | 1 | 0 | 1 | 1 |
| 3005 | 1,2 | 2,3 | 2,3 | 1 | 0 | 0 | 1 |
| 0002 | 1,8 | 2,3 | 2,3 | 1 | 0 | 0 | 1 |
| 0206 | 9,6 | 2,9 | 3 | 1 | 0 | 0 | 1 |
| 0135 | 11 | | 3 | 1 | 0 | 0 | 1 |
| 0175 | 16 | 2,9 | 3 | 1 | 0 | 0 | 1 |
| 0005 | 25 | 2,3 | 2,3 | 1 | 0 | 0 | 1 |
| 0278 | 25,7 | 4 | 3 | 1 | 0 | 0 | 1 |
| 0001 | 127 | 2,3 | 2,3 | 1 | 0 | 0 | 1 |
| 0004 | 127 | 2,9 | 3 | 1 | 0 | 0 | 1 |
| 0037 | 127 | 2,9 | 3 | 1 | 0 | 0 | 1 |
| 0294 | 127 | 14 | 2,3 | 1 | 0 | 0 | 1 |
| 0108 | 17,5 | | 3 | 1 | | 0 | 1 |
| 28 | 0,6 | 69 | 330 | 0 | 1 | 1 | 1 |
| 0012 | 0,6 | 23 | 8,6 | 0 | 1 | 1 | 1 |
| 0013 | 0,6 | 27,7 | 3 | 0 | 1 | 0 | 1 |
| 6c | 0,6 | 2,8 | 35 | 0 | 0 | 1 | 1 |
| 18c | 0,6 | 2,3 | 15 | 0 | 0 | 1 | 1 |
| 53 | 0,6 | 2,3 | 17 | 0 | 0 | 1 | 1 |
| 0164 | 0,6 | 2,9 | 5,9 | 0 | 0 | 1 | 1 |
| 3007 | 0,6 | 2,3 | 2,3 | 0 | 0 | 0 | 0 |
| 9c | 0,6 | 2,3 | 2,3 | 0 | 0 | 0 | 0 |
| 13c | 0,6 | 2,3 | 2,3 | 0 | 0 | 0 | 0 |
| 47 | 0,6 | 2,3 | 2,3 | 0 | 0 | 0 | 0 |
| 17 | 0,6 | 2,3 | 2,3 | 0 | 0 | 0 | 0 |
| 18 | 0,6 | 2,3 | 2,3 | 0 | 0 | 0 | 0 |
| 0331 | 0,6 | 2,3 | 3 | 0 | 0 | 0 | 0 |
| 0063 | 0,6 | 2,9 | 3 | 0 | 0 | 0 | 0 |
| 0179 | 0,6 | 2,9 | 3 | 0 | 0 | 0 | 0 |
| 0213 | 0,6 | 2,9 | 3 | 0 | 0 | 0 | 0 |
| 0233 | 0,6 | 2,9 | 3 | 0 | 0 | 0 | 0 |
| 0081 | 0,6 | 2,9 | 14,4 | 0 | | 1 | 1 |

**Tabelle 4c: Wahrheitswerte für den Nachweis von Urease, 26kDa-Protein, und HSP60**

| | **Wahrheitswert** | | | |
|---|---|---|---|---|
| | **Urease** | **26kDa** | **HSP60** | **3-Kombination** |
| **Schwellenwert** | **1** | **15** | **5** | |
| **Spezifität** | **88%** | **90%** | **77%** | **75%** |
| **Sensitivität** | **57%** | **26%** | **41%** | **75%** |
| negativ-richtige | 35 | 36 | 17 | 30 |
| negativ-gesamt | 40 | 40 | 22 | 40 |
| positiv-richtige | 25 | 11 | 18 | 33 |
| positiv-gesamt | 44 | 42 | 44 | 44 |

| | | | | |
|---|---|---|---|---|
| Abkürzungen: 26kDa bedeutet 26 kDa-Protein; 3-Kombination bedeutet Kombination aller drei Antigene (Urease, 26kDa-Protein und HSP60) | | | | |

Als Fänger-Antikörper und Detektions-Antikörper wurden bei Urease HP8m/4H5 + HP16m/XG1 eingesetzt. Die Nachweisgrenze lag bei 0,075 ng/ml. Bei 26kDa-Protein wurden als Fänger-Antikörper HP15m/4H12 und als Detektions-Antikörper HP15m/3E8 eingesetzt. Die Nachweisgrenze lag bei 1,5 ng/ml. Bei HSP60 wurden als Fang-Antikörper HP18.1m/3F11 und als Detektions-Antikörper HP16m/2A5 + HP18.1m/4D9 eingesetzt. Die Nachweisgrenze lag bei 6 ng/ml.

### Ergebnisse

Die Tabellen 4a - 4c zeigen die Ergebnisse der Untersuchung von Stuhlproben für Urease, Alkylhydroperoxid-Reduktase und HSP60. Die letzte Spalte (3-Kombination) zeigt jeweils die Werte für den resuttierenden Wahrheitswert der Kombination aus allen drei Antigenen (Urease, 26kDa-Protein, HSP60).
Insgesamt wurden von 40 untersuchten Kategorie 0 Kontrollproben (Tabelle 4a) 30 als negativ identifiziert (Spezifität 75%), von 44 Kategorie 4 Proben (Tabelle 4b) wurden 33 als positiv identifiziert (Sensitivität 75%). In 6 von 44 Proben war der Nachweis von allen drei Antigenen möglich, in 25 von 44 Proben konnte Urease, in 11 von 42 Proben konnte Alkylhydroperoxid-Reduktase und in 18 von 44 Proben konnte HSP60 nachgewiesen werden. Für die Kombination aus allen drei Antigenen (Urease, 26kDa, HSP60) wurde somit eine Spezifität von 75% sowie eine Sensitivität von 75% erreicht (Tabelle 4c).

Tabelle 5 zeigt das Ergebnis der Untersuchung von 3 Proben, die aus dem Stuhl eines Patienten an drei verschiedenen Stellen entnommen wurden. Die Zahlenangaben entsprechen der gemessenen Extinktion bei 650nm dividiert durch die Extinktion einer Nullprobe, also dem Vielfachen des Hintergrunds (H). Es zeigte sich eine inhomogene Verteilung der nachgewiesenen Antigene.

**Tabelle 5: Verteilung H. pylori-spezifischer Antigene in Stuhlproben, die an verschiedenen Stellen des Stuhlgangs eines Infizierten entnommen wurden**

| **Detektiertes Antigen** | **β-Urease** | **26kDa-Protein** | **HSP60** |
|---|---|---|---|
| Probe 1 | 8,6 x H | 2,5 x H | 1 x H |
| Probe 2 | 11 x H | 1 x H | 1 x H |
| Probe 3 | 3,5 x H | 1 x H | 1 x H |

### Beispiel 10: Klonierung und Sequenzbestimmung der funktionellen variablen Bereiche von Immunoglobulinen aus Hybridom-Zellinien

Gesamt-RNA wurde aus Antikörper-produzierenden Hybridom-Zellinien nach Chomczynski (Chomczynski, 1987) isoliert.

Anschließend wurde die entsprechende cDNA nach Standard-Methoden synthetisiert (Sambrook et al., 1989)

Die DNA-Regionen, welche die kappa-leichte Kette sowie das schwere Kette-Fd-Segment (VH + CH1) der jeweiligen Antikörper kodieren wurden mittels PCR amplifiziert. Dabei kam das in Tabelle 6 aufgeführte Oligunukleotid-Primer Set zur Anwendung. Die aus den einzelnen Hybridom-Zellinien isolierte cDNA diente als Matrize.

Das eingesetzte Primer-Set führt zu je einer 5'*-Xhol* und einer 3'-*Spe*l Schnittstelle in den schwere Kette-Fd-Fragmenten sowie zu je einer 5'-Sacl und einer 3'-Xbal-Schnittstelle in den kappa-leichten Ketten. Zur PCR-Amplifikation der schwere Kette-Fd-kodierenden DNA-Fragmente wurden 11 verschiedene 5'-VH-Primer (MVH 1-8 und MULH1-3) jeweils kombiniert mit dem 3'-VH-Primer MlgG1. Zur Amplifikation der DNA-Fragmente, welche für die kappa leichten Ketten kodieren, wurden 11 verschiedene 5'-VK-Primer (MUVK 1-7 und MULK1-4) jeweils kombiniert mit dem 3'-VK-Primer 3'MUCK.

Das folgende Temperaturprogramm kam bei allen PCR-Amplifikationen zur Anwendung: anfängliche Denaturierung bei 94°C für 3 min, Denaturierung bei 94 °C für 25 s, primer-Anlagerung bei 52°C für 60 s, Polymerisation bei 72 °C für 90 s. Dieses Programm wurde für 40 Zyklen beibehalten, gefolgt von einer 10 min. abschließenden Vervollständigung der Fragmente bei 72°C.

Die Ergebnisse der PCR-Amplifikationen wurden mittels Agarose-Gel-Elektrophorese aufgetrennt und DNA-Banden des erwarteten Molekulargewichts isoliert. Die isolierten Banden wurden anschließend einem Restriktionsverdau unter Einsatz der Enzyme *Xhol* und *Spel* (schwere Ketten) bzw. *Sacl* und *Xbal* (leichte Ketten) unterzogen und die erhaltenenen Fragmente in den Plasmid-Vektor Bluescript KS (Stratagene) kloniert, nachdem dieser zunächst mit den Restriktionsenzymen *Xhol* und *Spel* bzw. *Sacl* und *Xbal* gespalten worden war.

Plasmid-Präparationen der klonierten schwere und leichte Ketten-Fragmente wurden daraufhin sequenzanalysiert. Für jede Hybridom-Zellinie wurden Sequenzen ausgewählt, die für funktionelle variable Bereiche der Immunglobulin schwere und leichte Kette (VH bzw. VL) kodieren. Auf diese Weise konnte für jede Hybridom-Zellinie genau ein funktioneller VH- und ein funktioneller VL-Bereich identifiziert werden. Die funktionellen VH- und VL-Sequenzen sind in Abbildungen 1-8 wiedergegeben. Klonierung und Sequenzierung wurden nach Standardmethoden durchgeführt (Sambrook et al., 1989).

**Tabelle 6: Liste der für die PCR-Amplifikation der funktionellen variablen Bereiche von schweren und leichten Immunglobulin-Ketten verwendet wurden**

| | |
|---|---|
| MVH1 | (GC)AG GTG CAG CTC GAG GAG TCA GGA CCT |
| MVH2 | GAG GTC CAG CTC GAG CAG TCT GGA CCT |
| MVH3 | CAG GTC CAA CTC GAG CAG CCT GGG GCT |
| MVH4 | GAG GTT CAG CTC GAG CAG TCT GGG GCA |
| MVH5 | GA(AG) GTG AAG CTC GAG GAG TCT GGA GGA |
| MVH6 | GAG GTG AAG CTT CTC GAG TCT GGA GGT |
| MVH7 | GAA GTG AAG CTC GAG GAG TCT GGG GGA |
| MVH8 | GAG GTT CAG CTC GAG CAG TCT GGA GCT |
| MULK1 | GGG GAG CTC CAC CAT GGA GAC AGA CAC ACT CCT GCT AT |
| MULK2 | GGG GAG CTC CAC CAT GGA TTT TCA AGT GCA GAT TTT CAG |
| MULK3 | GGG GAG CTC CAC CAT GGA GWC ACA KWC TCA GGT CTT TRT A |
| MULK4 | GGG GAG CTC CAC CAT GKC CCC WRC TCA GYT YCT KGT |
| MlgG1 | TAT GCA ACT AGT ACA ACC ACA ATC CCT GGG |
| MUVK1 | CCA GTT CCG AGC TCG TTG TGA CTC AGG AAT CT |
| MUVK2 | CCA GTT CCG AGC TCG TGT TGA CGC AGC CGC CC |
| MUVK3 | CCA GTT CCG AGC TCG TGC TCA CCC AGT CTC CA |
| MUVK4 | CCA GTT CCG AGC TCC AGA TGA CCC AGT CTC CA |
| MUVK5 | CCA GAT GTG AGC TCG TGA TGA CCC AGA CTC CA |
| MUVK6 | CCA GAT GTG AGC TCG TCA TGA CCC AGT CTC CA |
| MUVK7 | CCA GTT CCG AGC TCG TGA TGA CAC AGT CTC CA |
| MULH1 | GGG CTC GAG CAC CAT GGR ATG SAG CTG KGT MAT SCT CTT |
| MULH2 | GGG CTC GAG CAC CAT GRA CTT CGG GYT GAG CTK GGT TTT |
| MULH3 | GGG CTC GAG CAC CAT GGC TGT CTT GGG GCT GCT CTT CT |
| 3'MUCK | GCG CCG TCT AGA ATT AAC ACT CAT TCC TGT TGA A |

### Literatur:

**Coller & Coller,** 1983: Coller, H.A., Coller, B.S., Meth. Enzymol. 121:412-417
**Harlow & Lane,** 1988: Harlow, E., Lane, D., Antibodies: A laboratory manual, Cold Spring Harbour Laboratory, New York
**Kearney et al.,** 1979: Kearney, J. Immunol. 123: 1548-1550
**Laemmli,** 1970: Laemmli, E.K., Nature 227: 680-685
**Peters & Baumgarten,** 1990: Peters, J.H., Baumgarten, H. (Hrsg.), Monoklonale Antikörper, Springer Verlag, Berlin
**Fägerstam et al.,** 1990: Fägerstam, L.G. et al., J. Mol. Recognit. 3: 208-214
**Malmqvist,** 1996: Malmqvist, M., Methods 9: 525-532
**Eschweiler et al.,** 1993: Eschweiler, B., et al., Zentralbl. F. Bakt. 280: 73-85
Pharmacia Biotech, 1994: Monoclonal Antibody Purification Handbook
Chomczynski, 1987: Anal. Biochem. 162: 156-159.
Sambrook et al., 1989: Molecular cloning: A laboratory manual, Cold Spring Harbour Laboratory Press, second edition
Vaughan et al., 1998: Nature Biotechnology 16: 535-539
Orlandi et al., 1989: Proc. Natl. Acad. Sci USA 86: 3833-3837
Janeway & Travers, 1997: Immunologie, 2. Auflage, Spektrum Akademischer Verlag GmbH, Heidelberg
Osborne et al., 1997: Curr. Opin. Chem. Biol. 1: 5-9
Stull und Szoka, 1995: Pharm. Res. 12: 465-483

### Annex zu den Anmeldungsunterlagen - nachgereichtes Sequenzprotokoll

## Patentansprüche

1. Verfahren zum Nachweis einer Infektion eines Säugers mit einem Säure-resistenten Mikroorganismus, wobei man
(a) eine Stuhlprobe des Säugers mit mindestens zwei verschiedenen monoklonalen Antikörpern, Fragmenten oder Derivaten davon oder Aptameren unter Bedingungen inkubiert, die eine Komplexbildung von Antigenen aus dem Säure-resistenten Mikroorganismus mit den Antikörpern, Fragmenten oder Derivaten davon oder den Aptameren erlauben, und wobei
(aa) der erste monoklonale Antikörper oder das Fragment oder Derivat davon oder das erste Aptamer ein Epitop des ersten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert;
(ab) der zweite monoklonale Antikörper oder das Fragment oder Derivat davon oder das zweite Aptamer ein vom Epitop des ersten Antigens verschiedenes Epitop eines zweiten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert,
wobei die Teile der Säuger gemäß (aa) und gemäß (ab) überlappen können und in der Summe im wesentlichen die Gesamtanzahl der infizierten Säuger ausmachen; und
(b) die Bildung mindestens eines Antigen-Antikörperkomplexes oder Antigen-Aptamerkomplexes gemäß (aa) oder (ab) nachweist.

2. Verfahren nach Anspruch 1, wobei der Mikroorganismus ein Säure-resistentes Bakterium ist.

3. Verfahren nach Anspruch 2, wobei das Säure-resistente Bakterium ein Bakterium der Gattung Helicobacter oder der Gattung Mycobacterium oder der Gattung Campylobacter ist.

4. Verfahren nach Anspruch 3, wobei das Bakterium ein Bakterium der Spezies *Helicobacter pylori oder Helicobacter hepaticus* oder der Spezies *Mycobacterium tuberculosis* oder der Spezies *Campylobacter jejuni* oder *Campylobacter pylori* ist.

5. Verfahren nach einem der Ansprüche 1 bis 4, wobei das Epitop des ersten Antigens ein Epitop einer Urease und das Epitop des zweiten Antigens ein Epitop eines Hitzeschockproteins, einer Alkylhydroperoxid-Reduktase oder des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase) ist.

6. Verfahren nach Anspruch 5, wobei die Urease die β-Urease von *H. pylori* ist.

7. Verfahren nach Anspruch 5 oder 6, wobei das Hitzeschockprotein ein Hsp60 ist.

8. Verfahren nach einem der Ansprüche 5 bis 7, wobei die Alkylhydroperoxid-Reduktase das 26kDa-Protein von *H. pylori* ist.

9. Verfahren insbesondere nach einem der Ansprüche 1 bis 8, wobei man
(a) eine Stuhlprobe des Säugers mit drei verschiedenen monoklonalen Antikörpern, Fragmenten oder Derivaten davon oder Aptameren unter Bedingungen inkubiert, die eine Komplexbildung von Antigenen aus dem Säure-resistenten Mikroorganismus mit den Antikörpern, Fragmenten davon oder Derivaten oder den Aptameren erlauben, und wobei
(aa) der erste monoklonale Antikörper oder das Fragment oder Derivat davon oder das erste Aptamer ein Epitop des ersten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert;
(ab) der zweite monoklonale Antikörper oder das Fragment oder Derivat davon oder das zweite Aptamer ein vom Epitop des ersten Antigens verschiedenes Epitop eines zweiten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; und
(ac) der dritte monoklonale Antikörper oder das Fragment oder Derivat davon oder das dritte Aptamer ein vom Epitop des ersten und zweiten Antigens verschiedenes Epitop eines dritten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert,
wobei die Teile der Säuger gemäß (aa), gemäß (ab) und gemäß (ac) überlappen können und in der Summe im wesentlichen die Gesamtanzahl der infizierten Säuger ausmachen, und
(b) die Bildung mindestens eines Antigen-Antikörperkomplexes oder eines Antigen-Aptamerkomplexes gemäß (aa), (ab) oder (ac) nachweist.

10. Verfahren nach Anspruch 9, wobei das Epitop des ersten Antigens ein Epitop einer Urease, vorzugsweise der β-Urease von *H. pylori,* das Epitop des zweiten Antigens ein Epitop eines Hitzeschockproteins, vorzugsweise von Hsp60 aus *H. pylori* und das Epitop des dritten Antigens ein Epitop einer Alkylhydroperoxid-Reduktase, vorzugsweise des 26kDa-Proteins von *H. pylori* ist oder wobei das Epitop des zweiten oder dritten Antigens ein Epitop einer Alkylhydroperoxid-Reduktase oder des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase) ist.

11. Verfahren nach einem der Ansprüche 1 bis 10, wobei mindestens einer der monoklonalen Antikörper bzw. eines der Fragmente, Derivate oder Aptamere ein Konformationsepitop bindet.

12. Verfahren nach Anspruch 11, wobei sämtliche monoklonalen Antikörper bzw. Fragmente, Derivate oder Aptamere Konformationsepitope binden.

13. Verfahren zum Nachweis einer Infektion mit *Helicobacter pylori* im Stuhl eines Säugers, wobei man
(a) eine Stuhlprobe mit mindestens zwei verschiedenen monoklonalen Antikörpern, Fragmenten, Derivaten davon oder Aptameren unter Bedingungen inkubiert, die eine Antigen-Antikörper/Antigen-Aptamer-Komplexbildung erlauben, wobei
(aa) der erste monoklonale Antikörper, das Fragment, Derivat davon oder das erste Aptamer spezifisch β-Urease oder ein Fragment davon bindet;
(ab) der zweite monoklonale Antikörper, das Fragment, Derivat davon oder das zweite Aptamer spezifisch das 26kDa-Antigen oder ein Fragment davon bindet oder spezifisch Hsp60 oder ein Fragment davon bindet; und
(b) die Bildung mindestens eines Antigen-Antikörperkomplexes/Antigen-Aptamerkomlexes gemäß (aa) oder (ab) naehweist.

14. Verfahren zum Nachweis einer Infektion mit *Helicobacter pylori* im Stuhl eines Säugers, wobei man
(a) eine Stuhlprobe mit drei verschiedenen monoklonalen Antikörpern, Fragmenten, Derivaten davon oder Aptameren unter Bedingungen inkubiert, die eine Antigen-Antikörper/Antigen-Aptamer-Komplexbildung erlauben, wobei
(aa) der erste monoklonale Antikörper, das Fragment, Derivat davon oder das erste Aptamer spezifisch β-Urease oder ein Fragment davon bindet;
(ab) der zweite monoklonale Antikörper, das Fragment, Derivat davon oder das zweite Aptamer spezifisch Hsp60 oder ein Fragment davon bindet; und
(ac) der dritte monoklonale Antikörper, das Fragment, Derivat davon oder das dritte Aptamer spezifisch das 26kDa-Antigen oder ein Fragment davon bindet; und
(b) die Bildung mindestens eines Antigen-Antikörper/Antigen-AptamerKomplexes gemäß (aa), (ab) oder (ac) nachweist.

15. Verfahren nach einem der Ansprüche 7 bis 14, wobei der Hsp60-spezifische Antikörper der von dem bei der Deutschen Sammlung von Mikroorganismen und Zellkulturen (DSMZ) am 23. Juni 1998 nach den Statuten des Budapester Vertrages unter der Hinterlegungsnummer DSM ACC2356 hinterlegten Hybridom HP16m/2A5-E6-E5 produzierte Antikörper ist.

16. Verfahren nach einem der Ansprüche 8 bis 15, wobei der 26kDa-Antigen-spezifische Antikörper der von dem bei der Deutschen Sammlung von Mikroorganismen und Zellkulturen (DSMZ) am 23. Juni 1998 nach den Statuten des Budapester Vertrages unter der Hinterlegungsnummer DSM ACC2355 hinterlegten Hybridom HP15m/3E8-D9-D6 produzierte Antikörper ist.

17. Verfahren nach einem der Ansprüche 6 bis 16, wobei der β-Ureasespezifische Antikörper der von einem der bei der Deutschen Sammlung von Mikroorganismen und Zellkulturen (DSMZ) am 23. Juni 1998 nach den Statuten des Budapester Vertrages unter den Hinterlegungsnummern DSM ACC2360 oder DSM ACC2362 hinterlegten Hybridomen HP8m/4H5-D4-C9 oder HP9.1m/3C2-F8-E2 produzierte Antikörper ist.

18. Verfahren nach einem der Ansprüche 7 bis 17, wobei die schwere Kette des ein Hsp60-Epitop bindenden Antikörpers mindestens eine der folgenden CDRs, vorzugsweise die CDR3 und weiter bevorzugt alle drei folgenden CDRs aufweist:
CDR1: GFSLSRYSVH
CDR2: MIWGGGSTDYNSGLKS
CDR3: NMGGRYPDYFDY

19. Verfahren nach Anspruch 18, wobei die die schwere Kette des Antikörpers kodierende DNA-Sequenz mindestens eine der folgenden CDRs, vorzugsweise die CDR3 und weiter bevorzugt alle drei folgenden CDRs aufweist:
CDR1: GG GTTCTCATTA TCCAGATATA GTGTACAC
CDR2: ATGATATGGG GTGGTGGAAG CACAGACTAT AATTCAGGTC TCAAATCC
CDR3: AATATG GGGGGTAGGT ACCCGGACTA CTTTGACTAC

20. Verfahren nach einem der Ansprüche 7 bis 19, wobei die leichte Kette des ein Hsp60-Epitop bindenden Antikörpers mindestens eine der folgenden CDRs, vorzugsweise die CDR3 und weiter bevorzugt alle drei folgenden CDRs aufweist:
CDR1: RASKSVSTSGYSYIH
CDR2: LASNLES
CDR3. QHSRELPLT

21. Verfahren nach Anspruch 20, wobei die die leichte Kette des Antikörpers kodierende DNA-Sequenz mindestens eine der folgenden CDRs, vorzugsweise die CDR3 und weiter bevorzugt alle drei folgenden CDRs aufweist:
CDR1: A GGGCCAGCAA GAGTGTCAGT ACATCTGGCT ATAGTTACAT ACAC
CDR2: C TTGCATCCAA CCTAGAATCT
CDR3: CAGC ACAGTAGGGA GCTTCCGCTC ACG.

22. Verfahren nach einem der Ansprüche 8 bis 21, wobei die schwere Kette des ein 26kDa-Protein bindenden Antikörpers mindestens eine der folgenden CDRs, vorzugsweise die CDR3 und weiter bevorzugt alle drei folgenden CDRs aufweist:
CDR1: GFTFNSYAMY
CDR2: RIRSKSDNYATYYANSVKD
CDR3: DHDKFPFYYALDY

23. Verfahren nach Anspruch 22, wobei die die schwere Kette des Antikörpers kodierende DNA-Sequenz mindestens eine der folgenden CDRs, vorzugsweise die CDR3 und weiter bevorzugt alle drei folgenden CDRs aufweist:
CDR1: GG TTTCACCTTC AATTCCTATG CCATGTAC
CDR2: CGCATAAGAA GTAAAAGTGA TAATTATGCA ACATATTATG CCAATTCAGT GAAAGAC
CDR3: GATCATG ATAAGTTTCC TTTTTACTAT GCTCTGGACT AC

24. Verfahren nach einem der Ansprüche 8 bis 23, wobei ein Antikörper, Fragment oder Derivat davon eingesetzt wird, dessen leichte Kette des ein 26kDa-Protein bindenden Antikörpers mindestens eine der folgenden CDRs, vorzugsweise die CDR3 und weiter bevorzugt alle drei folgenden CDRs aufweist:
CDR1: TASSSVSSSYLH
CDR2: STSNLAS
CDR3: HQYHRSPPT

25. Verfahren nach Anspruch 24, wobei die die leichte Kette des Antikörpers kodierende DNA-Sequenz mindestens eine der folgenden CDRs, vorzugsweise die CDR3 und weiter bevorzugt alle drei folgenden CDRs aufweist:
CDR1: A CTGCCAGCTC AAGTGTGAGT TCCAGTTACT TGCAC
CDR2: AGCACTTCCA ACCTGGCTTC T
CDR3: CAC CAGTATCATC GTTCCCCACC GACG

26. Verfahren nach einem der Ansprüche 6 bis 25, wobei die schwere Kette des ein Epitop der β-Urease bindenden Antikörpers mindestens eine der folgenden CDRs, vorzugsweise die CDR3 und weiter bevorzugt alle drei folgenden CDRs aufweist:
CDR1: GFTFSSHFMS
CDR2: SISSGGDSFYPDSLKG
CDR3: DYSWYALDY
oder:
CDR1: GYAFSTSWMN
CDR2: RIYPGDGDTNYNGKFKG
CDR3: EDAYYSNPYSLDY

27. Verfahren nach Anspruch 26, wobei die die schwere Kette kodierende DNA-Sequenz des Antikörpers mindestens eine der folgenden CDRs, vorzugsweise die CDR3 und weiter bevorzugt alle drei folgenden CDRs aufweist:
CDR1: GG CTACGCATTC AGTACCTCCT GGATGAAC
CDR2: CGGATTTATC CTGGAGATGG AGATACTAAC TACAATGGGA AGTTCAAGGG C
CDR3: GAG GATGCCTATT ATAGTAACCC CTATAGTTTG GACTAC
oder:
CDR1: GG ATTCACTTTC AGTAGCCATT TCATGTCT
CDR2: TCCATTAGTA GTGGTGGTGA CAGTTTCTAT CCAGACAGTC TGAAGGGC
CDR3: GACTAC TCTTGGTATG CTTTGGACTA C

28. Verfahren nach einem der Ansprüche 6 bis 27, wobei die leichte Kette des ein Epitop der β-Urease bindenden Antikörpers mindestens eine der folgenden CDRs, vorzugsweise die CDR3 und weiter bevorzugt alle drei folgenden CDRs aufweist:
CDR1: RASQSIGTRIH
CDR2: YGSESIS
CDR3: QQSNTWPLT
oder:
CDR1: HASQNINVWLS
CDR2: KASNLHT
CDR3: QQGRSYPLT

29. Verfahren nach Anspruch 28, wobei die die leichte Kette des Antikörpers kodierende DNA-Sequenz mindestens eine der folgenden CDRs, vorzugsweise die CDR3 und weiter bevorzugt alle drei folgenden CDRs aufweist:
CDR1: A GGGCCAGTCA GAGCATTGGC ACAAGAATAC AC
CDR2: TAT GGTTCTGAGT CTATCTCT
CDR3: CAACAA AGTAATACCT GGCCGCTCAC G
oder:
CDR1: C ATGCCAGTCA GAACATTAAT GTTTGGTTAA GC
CDR2: AAG GCTTCCAACT TGCACACA
CDR3: CAACAG GGTCGAAGTT ATCCTCTCAC G

30. Verfahren nach einem der Ansprüche 6 bis 29, wobei die Antikörper in den variablen Regionen der leichten und schweren Ketten die in den Figuren 1 und 2, den Figuren 3 und 4, den Figuren 5 und 6 oder den Figuren 7 und 8 dargestellten Aminosäuresequenzen aufweisen.

31. Verfahren nach einem der Ansprüche 6 bis 30, wobei die kodierenden Bereiche der variablen Regionen der leichten und schweren Ketten die in den Figuren 1 und 2, den Figuren 3 und 4, den Figuren 5 und 6 oder den Figuren 7 und 8 dargestellten DNA-Sequenzen aufweisen.

32. Verfahren nach einem der Ansprüche 1 bis 31, wobei mit der Stuhlprobe vor der Inkubation mit den Antikörpern folgende Schritte durchgeführt werden: (a) Resuspendieren der Stuhlprobe 1:3 bis 1:25, vorzugsweise etwa 1:10 in Resuspendierungspuffer und (b) Mischen auf einem Vortexmixer.

33. Verfahren nach einem der Ansprüche 1 bis 32, wobei der Nachweis der Bildung des mindestens einen Antigen-Antikörperkomplexes/Antigen-Aptamerkomplexes in Schritt (b) mittels eines immunologischen Verfahrens erfolgt.

34. Verfahren nach einem der Ansprüche 1 bis 33, wobei der Nachweis der Bildung des mindestens einen Antigen-Antikörperkomplexes/Antigen-Aptamerkomplexes in Schritt (b) mittels ELISA, RIA, Western Blot oder eines immunchromatographischen Verfahrens erfolgt.

35. Verfahren nach Anspruch 33 oder 34, wobei im RIA oder im ELISA der gleiche Antikörper oder dessen Fragment oder Derivat oder das gleiche Aptamer zur Bindung an die Festphase wie zum Nachweis des Epitops eingesetzt wird.

36. Verfahren nach einem der Ansprüche 1 bis 35, wobei die Antikörper, Fragmente oder Derivate davon oder die Aptamere an einem Träger fixiert sind.

37. Verfahren nach einem der Ansprüche 1 bis 36, wobei der monoklonale Antikörper ein Maus-Antikörper ist.

38. Verfahren nach Anspruch 36, wobei das Trägermaterial des Trägers ein poröses Trägermaterial ist.

39. Verfahren nach Anspruch 36 oder 38, wobei das Trägermaterial ein Teststreifen ist.

40. Verfahren nach Anspruch 36, 38 oder 39, wobei das Trägermaterial aus Zellulose oder einem Zellulosederivat besteht.

41. Verfahren nach einem der Ansprüche 1 bis 40, wobei der Säuger ein Mensch ist.

42. Monoklonaler Antikörper, Fragment oder Derivat davon, der/das eine V-Region aufweist, die eine Kombination der in einem der Ansprüche 18 bis 29 dargestellten CDRs aufweist oder der von einem der in den Ansprüchen 15 bis 17 dargestellten Hybridomen produziert wird.

43. Monoklonaler Antikörper, Fragment oder Derivat davon nach Anspruch 42, der/das mindestens eine der in den Figuren 1 bis 8 dargestellten V-Regionen aufweist.

44. Monoklonaler Antikörper, Fragment oder Derivat davon nach Anspruch 42 oder 43, der ein Maus-Antikörper oder ein Fragment oder Derivat davon oder ein chimärer, vorzugsweise ein humanisierter Antikörper oder ein Fragment oder Derivat davon ist.

45. Aptamer, das dasselbe Epitop wie der monoklonale Antikörper, das Fragment oder Derivat davon nach einem der Ansprüche 42 bis 44 spezifisch bindet.

46. Epitop, das von einem monoklonaler Antikörper, Fragment oder Derivat davon nach einem der Ansprüche 42 bis 44 oder dem Aptamer nach Anspruch 45 spezifisch gebunden wird.

47. Antikörper, Fragment oder Derivat davon, der/das ein Epitop nach Anspruch 46 spezifisch bindet.

48. Diagnostische Zusammensetzung enthaltend mindestens zwei monoklonale Antikörper, Fragmente oder Derivate davon oder Aptamere wie in einem der vorstehenden Ansprüche definiert, gegebenenfalls fixiert an ein Trägermaterial.

49. Testvorrichtung zum Nachweis mindestens eines wie in einem der vorstehenden Ansprüchen definierten Epitops, umfassend
(a) mindestens zwei monoklonale Antikörper, Fragmente oder Derivate davon oder Aptamere wie in einem der vorstehenden Ansprüche definiert, fixiert an ein Trägermaterial;
(b) eine Vorrichtung zur Aufbereitung und Analyse von Stuhlproben; und gegebenfalls
(c) ein Gemisch von mindestens zwei monoklonalen Antikörpern, Fragmenten oder Derivaten davon oder Aptameren.

50. Testvorrichtung, zum Nachweis mindestens eines wie in einem der vorstehenden Ansprüchen definierten Epitops, umfassend
(a) mindestens zwei monoklonale Antikörper, Fragmente oder Derivate davon oder Aptamere wie in einem der vorstehenden Ansprüche definiert, wobei die Antikörper, Fragmente oder Derivate davon oder Aptamere konjugiert sind mit kolloidalem Gold, Latexpartikeln oder anderen farbgebenden Partikeln, deren Größe typischerweise im Bereich zwischen 5nm und 100nm, vorzugsweise zwischen 20nm und 60nm liegt;
(b) eine Vorrichtung zur Aufbereitung und Analyse von Stuhlproben; und gegebenfalls
(c) ein Gemisch von mindestens zwei monoklonalen Antikörpern, Fragmenten oder Derivaten davon oder Aptameren.

51. Kit enthaltend
(a) mindestens zwei monoklonale Antikörper, Fragmente oder Derivate davon oder Aptamere wie in einem der vorstehenden Ansprüche definiert, gegebenenfalls fixiert an ein Trägermaterial; gegebenenfalls
(b) eine Vorrichtung zur Aufbereitung und Analyse von Stuhlproben; und gegebenfalls
(c) ein Gemisch von mindestens zwei monoklonalen Antikörpern, Fragmenten oder Derivaten davon oder Aptameren.

52. Zusammensetzung, vorzugsweise Arzneimittel enthaltend mindestens einen der vorstehend beschriebenen Antikörper oder eines der vorstehend beschriebenen Fragmente, Derivate oder Aptamere, gegebenenfalls in Kombination mit einem pharmazeutisch verträglichen Träger und/oder Verdünnungsmittel.

53. Packung enthaltend die diagnostische Zusammensetzung nach Anspruch 48, die Testvorrichtung nach Anspruch 49, 50 oder den Kit nach Anspruch 51.
